# EUROPEAN PATENT APPLICATION

(11) **EP 0 714 907 A1**
(43) Date of publication of application: **05.06.1996**
(21) Application number: 95118193.2
(22) Date of filing: 18.11.1995
(51) Int. Cl.: C07H 21/02, C07H 19/073, C07H 19/173, A61K 31/70

(54) **Oligoribonucleotides with amide backbone**

(30) Priority: 30.11.1994 US 347541
(71) Applicant: F. HOFFMANN-LA ROCHE AG, CH-4002 Basel (CH)
(72) Inventor: Li, Wen-Ren, Nutley, New Jersey 07110 (US); Tam, Steve Yik-Kai, West Caldwell, New Jersey 07006 (US)
(74) Representative: Mezger, Wolfgang, Dr.

(57) **Abstract**

Oligomers having amide linkages of the following formula: 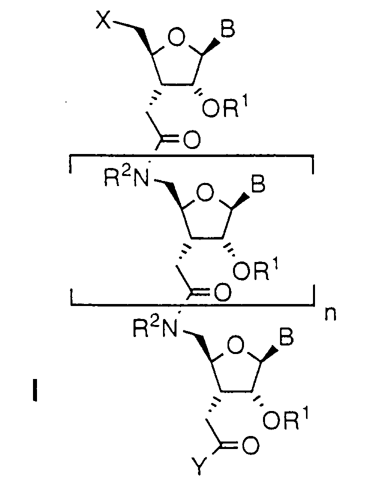
wherein R¹ is H, C₁₋₄ alkyl, C₁₋₁₈-acyl or hydroxy-lower alkyl; R² is H, aralkyl, or C₁₋₄ alkyl; B is a nucleoside base residue selected from the group consisting of adenine, cytosine, guanine, thymine, uracil; X is H, OR¹, NHR² or NH-acyl; Y is OR² or NHR²; and n is a number from 5 through 25, and salts thereof.

SEQUENCE LISTING

(1) GENERAL INFORMATION:

(i) APPLICANT:
(A) NAME: F.HOFFMANN-LA ROCHE AG
(B) STREET: Grenzacherstrasse 124
(C) CITY: Basle
(D) STATE: BS
(E) COUNTRY: Switzerland
(F) POSTAL CODE (ZIP): CH-4002
(G) TELEPHONE: 061 - 688 39 43
(H) TELEFAX: 061 - 688 13 95
(I) TELEX: 962292/965542 hlr ch

(ii) TITLE OF INVENTION: OLIGORIBONUCLEOTIDES WITH AMIDE-LINKED
BACKBONES

(iii) NUMBER OF SEQUENCES: 6

(iv) COMPUTER READABLE FORM:
(A) MEDIUM TYPE: Floppy disk
(B) COMPUTER: Apple Macintosh
(C) OPERATING SYSTEM: System 7.1 (Macintosh)
(D) SOFTWARE: Word 5.1

(2) INFORMATION FOR SEQ ID NO:1:

(i) SEQUENCE CHARACTERISTICS:
(A) LENGTH: 13 base pairs
(B) TYPE: nucleic acid
(C) STRANDEDNESS: single
(D) TOPOLOGY: linear

(ii) MOLECULE TYPE: other nucleic acid

(iii) HYPOTHETICAL: NO

(iv) ANTI-SENSE: YES

(xi) SEQUENCE DESCRIPTION: SEQ ID NO:1:

AGAGAGAGGA AGA 13

(2) INFORMATION FOR SEQ ID NO:2:

(i) SEQUENCE CHARACTERISTICS:
(A) LENGTH: 13 base pairs
(B) TYPE: nucleic acid
(C) STRANDEDNESS: single
(D) TOPOLOGY: linear

(ii) MOLECULE TYPE: other nucleic acid

(iii) HYPOTHETICAL: NO

(iv) ANTI-SENSE: YES

(xi) SEQUENCE DESCRIPTION: SEQ ID NO:2:

AGAAGGAGAG AGA 13

(2) INFORMATION FOR SEQ ID NO:3:

(i) SEQUENCE CHARACTERISTICS:
(A) LENGTH: 13 base pairs
(B) TYPE: nucleic acid
(C) STRANDEDNESS: single
(D) TOPOLOGY: linear

(ii) MOLECULE TYPE: other nucleic acid

(iii) HYPOTHETICAL: NO

(iv) ANTI-SENSE: NO

(xi) SEQUENCE DESCRIPTION: SEQ ID NO:3:

TCTCTCTCCT TCT 13

(2) INFORMATION FOR SEQ ID NO:4:

(i) SEQUENCE CHARACTERISTICS:
(A) LENGTH: 13 base pairs
(B) TYPE: nucleic acid
(C) STRANDEDNESS: single
(D) TOPOLOGY: linear

(ii) MOLECULE TYPE: other nucleic acid

(iii) HYPOTHETICAL: NO

(iv) ANTI-SENSE: YES

(xi) SEQUENCE DESCRIPTION: SEQ ID NO:4:

AGAAGAAGAG AGA 13

(2) INFORMATION FOR SEQ ID NO:5:

(i) SEQUENCE CHARACTERISTICS:
(A) LENGTH: 13 base pairs
(B) TYPE: nucleic acid
(C) STRANDEDNESS: single
(D) TOPOLOGY: linear

(ii) MOLECULE TYPE: other nucleic acid

(iii) HYPOTHETICAL: NO

(iv) ANTI-SENSE: YES

(xi) SEQUENCE DESCRIPTION: SEQ ID NO:5:

AGAAGCAGAG AGA 13

(2) INFORMATION FOR SEQ ID NO:6:

(i) SEQUENCE CHARACTERISTICS:
(A) LENGTH: 13 base pairs
(B) TYPE: nucleic acid
(C) STRANDEDNESS: single
(D) TOPOLOGY: linear

(ii) MOLECULE TYPE: other nucleic acid

(iii) HYPOTHETICAL: NO

(iv) ANTI-SENSE: YES

(xi) SEQUENCE DESCRIPTION: SEQ ID NO:6:

AGAAGUAGAG AGA 13

## Description

The highly specific interaction between synthetic oligonucleotides and RNA or single-stranded DNA has led to many applications in molecular biology. Oligonucleotides are used as hybridization probes for cloning, diagnostic assays, and as indispensable primer reagents in the polymerase chain reaction (PCR) technology. The specificity of binding is governed by the Watson Crick base-pairing rule. This feature allows the design of an oligonucleotide reagent to be fairly straightforward, requiring only a knowledge of the target RNA or DNA sequence. As a further extension of this attractive principle, binding of an oligonucleotide with its complementary mRNA sequence has been studied as a new tool for inhibiting the translation process of a specific protein product. In 1978, Zamecnik and Stephenson demonstrated the usefulness of the approach in the inhibition of Rous Sarcoma Virus development in infected chicken fibroblasts (Zamecnik, P.C.; Stephenson, M.L. *Proc. Natl Acad. Sci. U.S.A.* **1978** *75*, 280) This pioneering work has led to a blossoming of studies in the field and generated enormous interest in developing this concept for therapeutic applications. Since the oligonucleotide agent used is complementary (anti) to the sense of the genetic message contained in the mRNA target, this new approach was dubbed "antisense DNA" technology.

Phosphorothioates are widely known antisense compounds. These are backbone analogs in which one oxygen in the phosphodiester group is replaced by a sulfur. This conservative modification renders the molecules nuclease resistant and allows them to exhibit biological activities in cell cultures and in animal models. Phosphorothioate antisense drugs have entered clinical trials for evaluation as antiviral or anticancer agents.

However, while the potential of phosphorothiate antisense drugs remains promising, there is a need for new types of antisense agents due to side effects. Specifically, non-antisense side activities are exhibited by the phosphorothioates because their polythioate backbones are ionic. These side activities affect protein binding (Stein, C.A.; Narayanan, R. *Current Opinion in Oncology*, 6 (1994) 587), activation of immune cells and transcription factor(s) (Branda, R.F.; Moore, A.L.; Mathews, L.; McCormack, J.J.; Zon, G. *Biochem*. *Pharmacol*. **1993**, *8*, 33; and McIntyre, K.W. et al. *Antisense Res*. *Devel*, **1993**, *3*, 309), and RNase H cleavage of non-target sequences due to partial complementary binding (Giles, R.V.; Spiller, D.G.; Tidd, D.M. *Anticancer Drug Design* **1993**, *8*, 33).

To reduce non-antisense activities due to ionic backbones, oligonucleotides incorporating a few modified residues in which the phosphodiester linkage has been replaced with an amide have been synthesized (Burgess, K.; Gibbs, R.A.; Metzker, M.L.; Raghavachari, R. *J..C.S. Chem*. *Commun*. **1994**, 915; Chur, A.; Holst, B.; Dahl, O.; Valentin-Hansen, P.; Pedersen, E.B. *Nucleic Acid Res*. **1993**, *21*, 5179; Idziak, I.; Just, G.; Damha, M.; Giannaris, P. *Tet. Lett*. **1993**, *34*, 5417; De Mesmaeker,A.; Waldner, A.; Lebreton, J.; Hoffmann, P.; Fritsch, V.; Wolf, R.; Freier, S. *Angew*. *Chem*. *Intl*. *Ed.* Engl. **1994**, *33*, 226). Incorporation of these modified linkages into a single stranded DNA molecule usually results in a lowering of binding affinity with either a complementary RNA or DNA. However, no example of a total replacement of the phosphodiester groups by amide groups has been reported.

Another type of analog with higher binding affinity is the Peptide Nucleic Acid (PNA) (Nielsen, P.; Egholm, M.; Berg, R.; Buchardt, O. *Science*, **1991**, *254*, 1497). These molecules contain the familiar nucleic acid bases-adenine, guanine, thymine and cytosine-linked to a peptide backbone rather than the sugar-phosphate backbone. Although this type of compound has high binding affinity with DNA and RNA, this type of compound also shows poor specificity by binding to antiparallel as well as to parallel complementary sequences of RNAs. Moreover, the poor water solubility of these molecules has become a drawback to their practical application.

Thus, there is a need for an antisense molecule with a backbone linkage having several characteristics, namely the linkage should be stable to enzymatic cleavage, should be neutral to avoid side effects associated with polyanionic structures, should have acceptable affinity and specificity in its binding with RNA, and should have a desirable physical chemical properties, e.g. water solubility'. The antisense molecules of this invention meet these requirements. In contrast to existing art, antisense molecules of this invention are a new class of oligoribonucleotides in which the entire phosphodiester-linked backbone is replaced by an amide-linked backbone.

The present invention provides a new type of oligomer (oligoribonucleotide) which is composed of modified ribonucleoside monomer units connecting by amide linkages. Synthesis of these compounds can be achieved by oligomerization of monomeric intermediates which are also part of this invention, using for example solid phase or solution coupling methodology. Due to the specific structures of this invention, the antisense molecules disclosed herein have the advantages of stability and water solubility, and avoid ionic side effects and nonspecific protein binding.

In particular, the present invention is directed to an oligomer having the structure 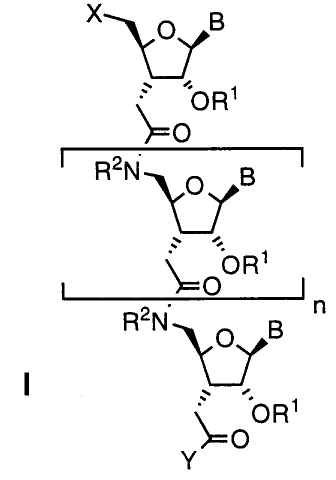
In formula I, R¹ can be H, lower alkyl having one to four carbon atoms (C₁-C₄), acyl having one to eighteen carbon atoms (C₁-C₁₈), or hydroxy-lower alkyl; R² is H, aralkyl or lower alkyl having one to four carbon atoms (C₁-C₄); B is a nucleoside base residue selected from the group consisting of adenine, cytosine, guanine, thymine and uracil; X can be H, OR¹, NHR² or NH-acyl; Y can be OR¹ or NHR²; and n can be any number from 5 through 25.

This oligomer is capable of binding to a selected mRNA sequence. Preferably, said oligonucleotide compound has a sequence of bases complementary to a selected RNA sequence. Also included are salts of the compounds of this invention.

R¹ may specifically be methyl, acetyl, or hydroxyethyl. In a specific embodiment R² is H, X is H, OR¹ or NHR² and R¹, B and Y are as defined in formula I above. In a preferred compound R¹ and R² are both H or R¹ is methyl and R² is H.

In a preferred combination X is NH₂ or NH-acyl, Y is OH or NH₂, R¹ is H or acetyl, R² is H and n is 6-13.

Moreover, an oligomer having the above structure but having a suitably protected nucleoside base residue (as defined below as B') in the B position and where m (instead of n) is 0 through 5 is also an object of this invention, in particular where m is 0, 1 or 2. Such oligomers are useful as intermediates for oligomers where n is 5 through 25.

Oligomers of Formula I are composed of modified ribonucleoside monomers connected by amide linkages. The oligomers of this invention are useful in any application involving oligomers, in particular because of enhanced stability over oligomers with unmodified linkages, and enhanced specificity and water solubility over oligomers with modified linkages of other types. In addition, it is possible by means of this invention to obtain oligomers all of whose component nucleosides are connected by amide linkages.

Because of their amide linkage, the present oligomers can be assembled into antisense compounds which are stable, water soluble, and in particular nuclease resistant. Thus, the oligomers of this invention are useful as antisense therapeutics which bind to a target mRNA to block or decrease the production of a target protein. Antisense therapeutics are used as described in Akhtar and Ivinson, *Nature Genetics*, **1993** *4*, 215. As an example of desighing an antisense oligomers, a target protein may be selected which causes or contributes to an undesirable condition. The nucleotide sequence of the gene or mRNA corresponding to the protein is then obtained by known methods, and oligomers complementary to part of this sequence may then be designed. The mRNA sequence may be determined on the basis that the mRNA encodes a protein whose translation is desired to be blocked in order to reduce or eliminate production of that protein to alleviate undesired effects caused in a subject by the presence of the protein. mRNA sequences which are not already known may be determined by translating the sequence of the encoded protein using the genetic code. If the protein sequence is not known, then it may be determined by isolating and sequencing the protein by known techniques. Alternatively, the mRNA or DNA encoding the protein may be identified, isolated, and sequenced by known methods.

A preferred size for such oligomers is in the range of about 7 to about 27, especially about 8 to about 15 monomers (n = 7-13).

These antisense oligomers, when made by the method of this invention as provided below to have the amide linkages of this invention, form stable antisense compounds which may then be administered to alleviate the condition associated with the presence of the target protein. The oligomers of this invention are especially useful as antisense compounds for treatment of conditions related to the production of undesired or excessive proteins whether native or foreign, and are also useful to block the proliferation of viruses or cancer cells. Antisense oligomers are designed to be complementary to the mRNA of a target gene, and bind to the RNA to prevent its translation, consequently reducing or preventing synthesis of the protein encoded by the target gene. Therefore, the ability to bind stably to nucleic acid under physiological conditions indicates antisense utility. In addition, an antisense compound must have appropriate sequence binding specificity in order to bind to the specific mRNAs of a target gene. Finally, antisense compounds must be sufficiently soluble to be physiologically effective and reach the target mRNA. These oligomers are also useful as PCR primers for use in PCR reactions, and have the advantage of stable and specific binding in PCR, and are also useful as probes in diagnostic tests employing nucleic acid hybridization.

The 3'- and 5'-terminal residues of the oligomers of this invention may be modified in any conventional way to make the compound more compatible with solid phase synthesis or to confer desirable physical chemical properties.

A specific application for the oligomers with linkages of this invention is in the antisense treatment of hyperpigmentation. Hyperpigmentation results from overproduction of the enzyme tyrosinase in melanocytes (pigment cells). Adding an oligomer of this invention which has the sequence corresponding to relevant portions of the mRNA encoding tyrosinase (Müller, G.; Ruppert, S.; Schmid, E.; Schutz, G. *EMBO* **1988**, *7*, 2723-30) inhibits tyrosinase production, thus eliminating hyperpigmentation in affected cells (Ando, S.; Ando, O.; Suemoto, Y.; Mishima, Y. *J. Invest. Dermatol.* **1993**, *100*, 1505-1555) (Kuzumaki, T.; Matsuda, A.; Wakamatsu, K.; Ito, S.; Ishikawa, K. *Expt. Cell Res.* **1993**, *207*, 33-40). The efficacy of an antisense oligomer for reducing hyperpigmentation may be determined using a cell-based antisense assay, for example the assay of Example 11.

By lower alkyl is meant methyl, ethyl, propyl, or butyl. By aralkyl is meant aromatic substituted alkyl, such as benzyl. By acyl is meant an organic residue derived from an organic acid by removal of a hydroxyl group, for example an aliphatic or aromatic acid such as acetic or benzoic acid respectively, if desired in substituted form or a heteroaromatic acid, preferably comprising a 5 to 6 membered ring containing at least one of the heteroatoms O, S, or N, such as 2-furan-carboxylic acid or 2-pyridine-carboxylic acid. By hydroxy lower alkyl are meant groups such as hydroxyethyl, hydroxypropyl, wherein the hydroxy group may be free or protected by any conventional protecting group such as an acyl group or an ether forming group including silyl ethers.

The bases B of formula I may include any combination of the known natural or modified pyrimidine and purine nucleotide bases. Preferred nucleotide base residues are the natural residues of the bases adenine, cytosine, guanine, thymine, and uracil. Modified bases which are known in the art, such as 7-deazaadenine, 5-azacytosine, or 6-mercaptopurine, may also be used. The sequence of bases for an antisense oligomer is selected such that the oligomer presents a base sequence that is complementary to a selected mRNA.

Once the desired base sequence is obtained, the antisense oligomers of this invention may be prepared accordingly using intermediates of this invention. These intermediates are prepared and then appropriately linked together by the method below, which is also part of this invention. The RNA binding property of the oligomer may be determined by conventional thermal melting techniques.

In another aspect, the present invention is directed to an intermediate compound having the formula: 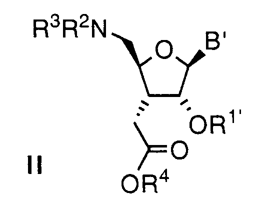
In formula II, R^{1'} is any conventional hydroxy protecting group such as a lower alkyl having one to four carbons (C₁₋₄), acyl having one to eighteen carbons (C₁₋₁₈), hydroxy lower alkyl, or benzyl; R² is H, aralkyl or (C₁-C₄)alkyl; R³ is an amino protecting group; and R⁴ is H or an acyl protecting group. B' is a suitably protected nucleoside base residue, such as N-benzoyl or N-acetylcytosine, N-benzoyladenine, thymine, uracil, or N-acetyl or N-isobutyrylguanine.

The compound of Formula II is a new intermediate which permits the synthesis of oligomers all of whose component nucleosides are connected by an amide linkage. Thus, these key intermediates of formula II and a method for producing compounds of Formula I using the intermediates of Formula II are an object of this invention.

In a preferred compound of formula II, R³ is 9-fluorenylmethoxycarbonyl, tert-butyloxycarbonyl, benzyloxycarbonyl, allyloxycarbonyl; triphenylmethyl, or 4,4'-dimethoxytrityl. In another preferred compound, R⁴ is tert-butyl, lower alkyl having one to four carbons, benzyl, phenyl, or 2-trimethylsilylethyl. Any combination of the preferred R³ and R⁴ forms a compound of this invention. Each compound of Formula II has as its nucleoside base a single base selected from the bases described above. In a specific embodiment R² is H.

Another intermediate compound of this invention has the formula: 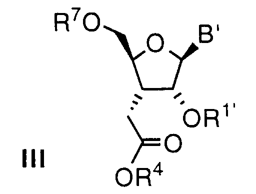
wherein R⁷ is a hydroxy protecting group; R⁴ is H, or an acyl protecting group; R^{1'} is any conventional hydroxy protecting group; and B' is a suitably protected nucleoside base residue.

The oligomer of formula I and their pharmaceutically acceptable salts can be used as a medicament, for example in the form of a pharmaceutical preparation.

The pharmaceutical preparations containing the antisense oligomers can be administered topically, e.g. intradermally or as an ointment on the skin, parenterally by injection or by gradual infusion over time. They can be administered intravenously, intraperitoneally, intramuscularly, subcutaneously or orally.

For the manufacture of a pharmaceutical preparation the oligomer of formula I and their pharmaceutically acceptable salts can be formulated with therapeutically inert carriers. Preparations for parenteral administration include sterile or aqueous or non-aqueous solutions, suspensions, and emulsions. Examples of non-aqueous carriers are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral carriers include sodium chloride solution, Ringer's dextrose, dextrose and sodium chloride, lactated Ringer's, or fixed oils. Intravenous vehicles include fluid and nutrient replenishers, electrolyte replenishers, such as those based on Ringer's dextrose, and the like. In particular, carriers capable of transporting oligomers to cells and into cells may be used, for example liposomes, PEGylated liposomes, or cationic lipids. Preservatives and other additives may also be present, such as anti-microbials, anti-oxidants, chelating agents, inert gases and the like. See, generally, *Remington's Pharmaceutical Science*, 18th Ed., Mack Eds., 1990.

In particular, the present invention includes a pharmaceutical composition which decreases the production of a target protein in a cell, which comprises any of the above-described compounds of Formula I in an amount effective to bind to the nucleic acid (for example mRNA) sequence encoding the target protein in said cell and decrease production of said protein, and a pharmaceutically acceptable carrier.

Medicaments containing a compound of formula I or a pharmaceutically acceptable salt thereof and a therapeutically acceptable carrier as well as a process for the manufacture of such medicaments are also objects of the present invention. This process comprises mixing a compound of formula I or a pharmaceutically acceptable salt thereof with a therapeutically inert carrier material and bringing the mixture into a galenical administration form.

As mentioned above, the compounds of formula I and their pharmaceutically acceptable salts can be used for decreasing the production of a target protein in a cell. The dose ranges for the administration of the antisense oligomers may be determined by those of ordinary skill in the art without undue experimentation. In general, appropriate dosages are those which are large enough to produce the desired effect. The dosage should not be so large as to cause adverse side effects, such as unwanted cross-reactions, anaphylactic reactions, and the like. Generally, the dosage will vary with the age, condition, sex and extent of disease in the patient, counter-indications, if any, immune tolerance and other such variables, to be adjusted by the individual physician.

The following Examples are provided to further describe the invention and are not intended to limit it in any way.

### SYNTHESIS OF MONOMERIC BUILDING BLOCKS

The most direct synthesis to the key intermediates of formula II is to employ ribonucleosides, eg. adenosine, uridine, guanosine, and cytidine, as the starting materials and then devise a method to replace the hydroxy group at C-3' with a carbon substituent. A similar strategy has been applied to the synthesis of the 2'-deoxy analogs with the use of free radical chemistry. (Fiandor, J.; Tam, S*. Tet. Lett.* **1990**, *31*, 597). However, in the ribonucleoside series, the same radical reaction does not furnish the desired stereospecificity.

The following new synthesis can specifically address this problem (General Scheme A). The starting material is commercially available D-xylose. After blocking of the OH groups at C-1 and C-2 as a ketal and the hydroxyl group at C-5 with a conventional protecting group for a primary alcohol (R⁷), eg. trityl or dimethyltertbutylsilyl, or preferably a sulfonyl group, such as toluenesulfonyl (Ts), methanesulfonyl (Ms) or trifluroromethylsulfonyl (Tf), the remaining OH group is then oxidized to a ketone (IV) by conventional methods, eg. DMSO/acetic anhydride or TEMPO. Olefination of the ketone in IV with a Wittig or Horner type reagent gives a mixture of *cis* and *trans* unsaturated esters (V). R⁴ is a conventional acyl protecting group. Among the acyl protecting groups which can be utilized in accordance with this invention are those where R⁴ taken together with its attached oxygen forms a hydrolyzable ester. The mixture V on catalytic hydrogenation furnishes the desired product VI with the *ribo* configuration. This high stereospecificity is controlled by the rigid conformation of the bicyclic system which allows only the exo-attack of the reagents. Hydrolysis of the ketal group in VI followed by acylation gives the glycosyl acetate VII, where R⁸ is an alkyl or aryl group, which can be directly used in a nucleoside coupling reaction by conventional methods. The procedures of using a silylated form of a suitably protected nucleo-base, a furanosylacetate and an activating agent such as trimethylsilyltriflate, stannic chloride or other Lewis acid catalysts in the synthesis of β-nucleosides are well known in the art. The intermediate VII can thus be used for the preparation of a variety of nucleosides VIII including those with the natural nucleo-bases, eg. cytosine, uracil, thymine, guanine and adenine, and those with unnatural bases, eg. 7-deazaadenine, 5-azacytosine, 6-mercaptopurine etc. Introduction of a nitrogen atom to the C-5' position of VIII is achieved readily by a displacement of the sulfonate with an azide or a phthalimide group. Subsequent reduction of the azide, eg. by a selective hydrogenation, or deblocking of the phthalimide group generates the amine IX.

For a compound with general formula X where R² is hydrogen, it can be easily obtain from a compound of formula IX by commonly known procedures which involve reagents such as 9-fluorenylmethyl chloroformate, N-(9-flurenylmethoxycarbonyloxyl)succimide, 2-tert-butoxycarbonyloxyimino)-2-phenylacetonitrile (BOC-ON), benzylchloroformate, or 4,4'-dimethoxytrityl chloride. For a compound with general formula X where R² is lower alkyl or aralkyl, it can be easily obtain from a compound of formula IX by reductive amination and amino protection procedure.

At this point, final removal of the ester group in X, eg. hydrogenation of a benzyl ester, furnishes the suitably protected monomeric building block (X, R⁴=H same as II, where R^{1'} =-COR⁸) for oligomer synthesis.

It should be pointed out that the C-5 amino function can be also introduced before the nucleoside formation step. Thus, XI is a more advanced common intermediate for various nucleosides with the general formula II, where R₁'=-COR⁸.

The compound of formula II where R^{1'}= lower alkyl, benzyl, protected alkanol (hydroxy-alkyl) and R⁴=H are prepared according to General Scheme B. Key to this route is the preparation of a 4-pentenyl glycoside intermediate XIV from a compound of formula VI (see General Scheme A). Accordingly, the intermediate VI is treated with 4-pentenyl-1-ol and TMS-triflate to give the lactone XII. The lactone can then be opened by treatment with a mixture of triethylamine-methanol-water to afford the hydroxy acid which is converted to a compound of general structure XIII by conventional alkylation methods. The pentenyl alkoxy group at the anomeric center in XIII can serve as a blocking group during this simple alkylation of the OH group at C-2 (eg., using a reaction of alkyl halide or protected derivative of ω-OH-alkyl halide in the presence of sodium hydride) but can be reactivated in the nucleoside coupling step. After adding an acid protecting group in XIII, such as a benzyl group, the nucleobase is introduced to the carbohydrate moiety which already contains the desired alkyl substituent group at C-2 (cf. formula XIV). This pentenyl glycoside can be also easily converted into other leaving groups such as an acetate or a halide by procedures well-known in the art. An example of the approach is shown in the Scheme 10.

A compound of formula XV can also be converted to the key intermediate XVI (II where R^{1'}= lower alkyl, benzyl, protected hydroxy-alkyl and R⁴=H) by procedures discussed in General Scheme A.

As a further variation, XIV can be converted first into XVII, a more advanced common intermediate, before the introduction of the nucleobase.

An alternate synthetic route to introduce alkyl groups at the 2'-OH position is shown in General Scheme C. In this route, the most versatile intermediate is XIX which is obtained by selective alkylation of the carboxylate group of XVIII, which in turn is prepared from hydrolysis of the ester groups in VIII. To avoid concomitant hydrolysis of the protecting groups of the nucleo-bases, enzymatic methods or a more selective base, such as potassium trimethylsilanoate, can be used. After introduction of the desired substituent to the 2'-OH position, compound XV can be converted to the key intermediate II where R^{1'}= lower alkyl, benzyl, protected hydroxy-alkyl and R⁴=H by procedures discussed in General Scheme A.

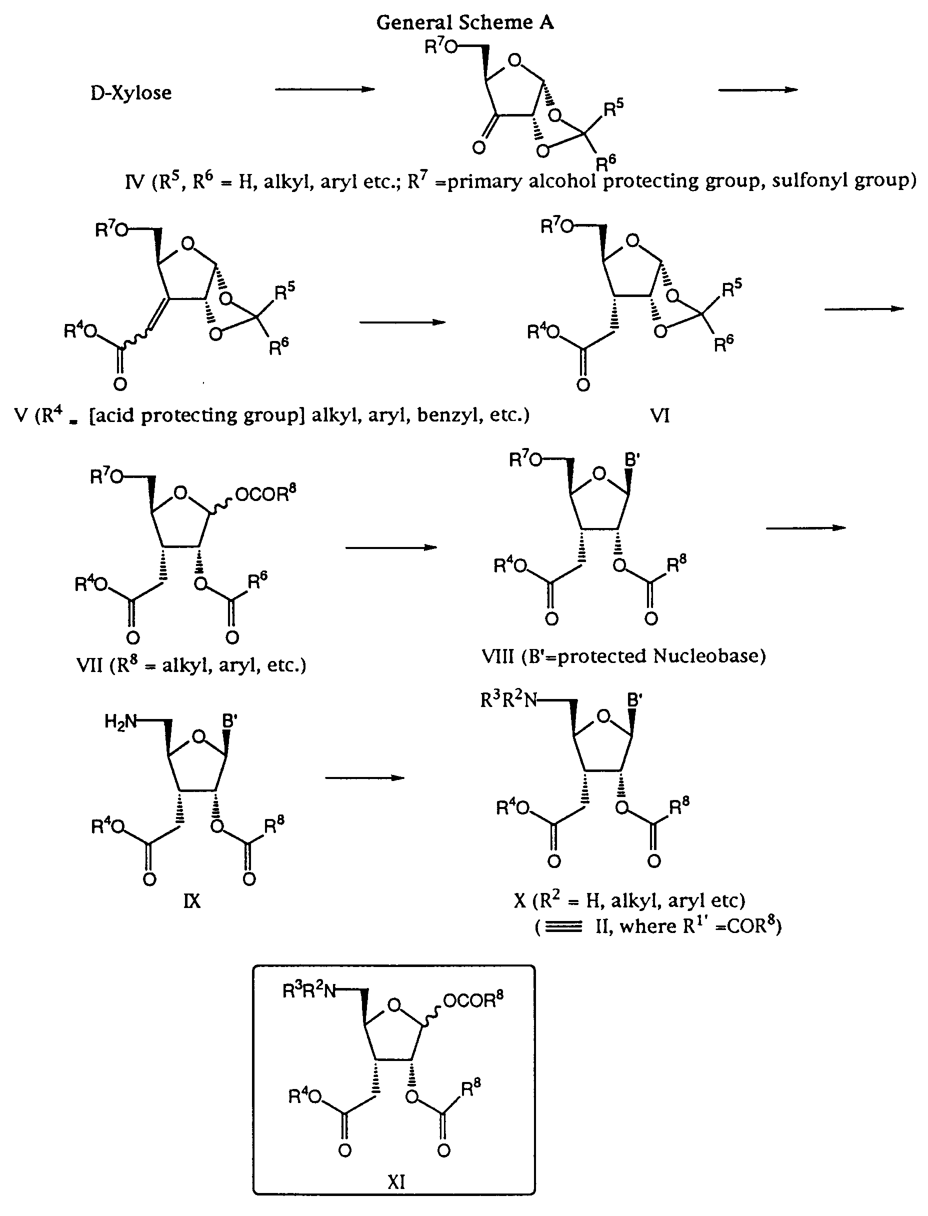

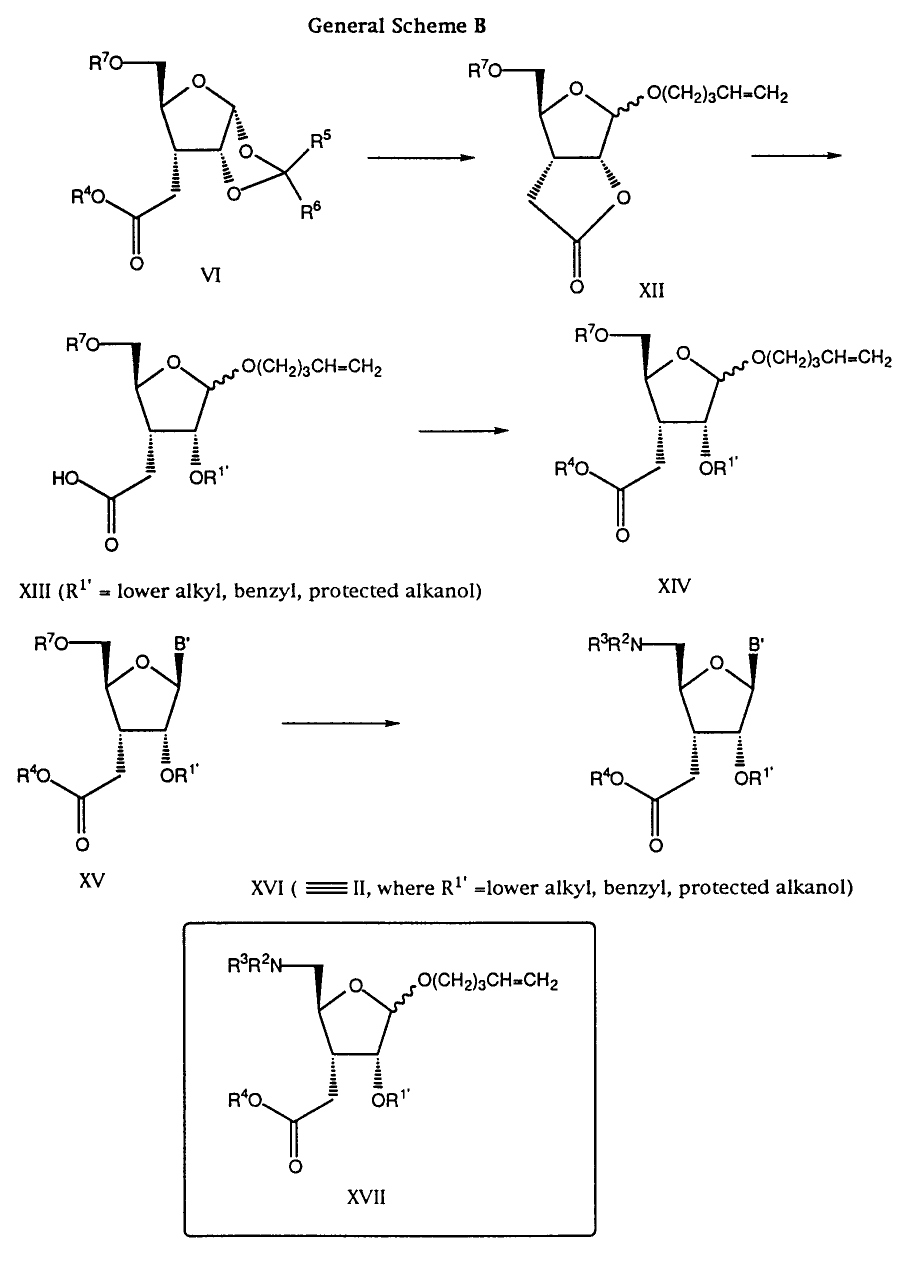

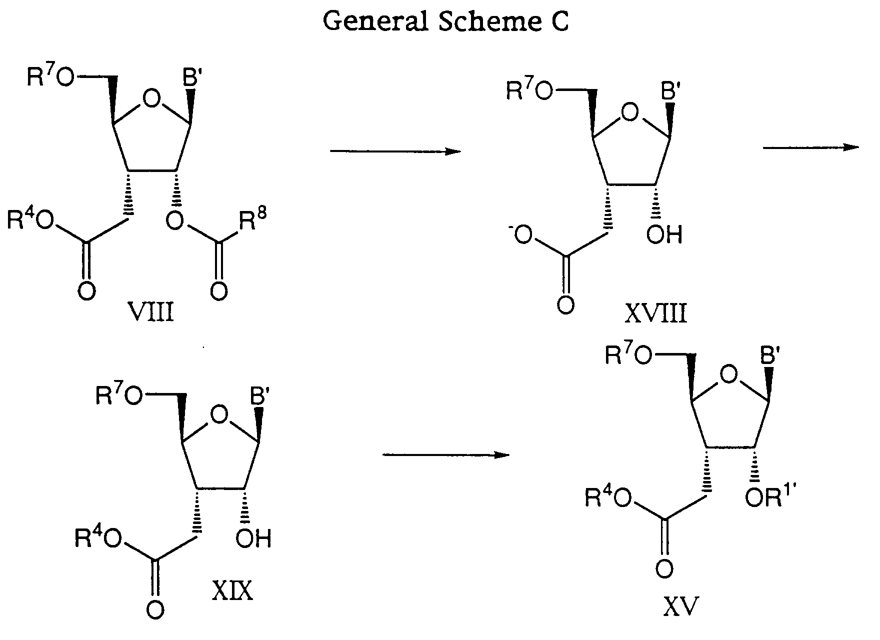

### SYNTHESIS OF OLIGOMERS

Assembly of the suitably protected monomeric building blocks of general formula II into oligomers can be carried out by the solid phase methodology or by the conventional solution phase coupling procedures. By choosing appropriate linker or resin, the oligomers can be synthesized either as free C-terminal carboxylic acids (Y= OH) or amides (Y = NH₂). For example with hydroxymethyl type resin (such as Wang resin), acidic cleavage will afford the oligomer as an acid; but aminolysis will give an amide. Alternatively, with the use of Rink acid linker and Rink amide linker, one can get an acid product or an amide product respectively by a simple mild acidic cleavage of the oligomer from solid support. These procedures are well known to people skilled in the art. For reference, a number of reviews on the solid phase procedures have been published including one that utilizes 9-fluorenylmethoxycarbonyl-protected amino acids (Fields, G. and Noble, R. *Int. J. Peptide Protein Res*. 35, **1990**, 161-214). Synthesis of dimers by the solution phase procedures are illustrated in Examples 5 and 6.

A detail procedure for the solid phase synthesis is illustrated in Example 8. In this example lysine was coupled to the C-terminal for analytical purpose.

For the preparation of oligomers of general formula I where X=H, OH, or OR¹, a monomeric building block of general formula III can be used in the last step of the oligomer assembly. Compounds of this general type (III) can be easily obtained from the VIII and XV (shown in General Scheme A and B respectively) by conventional methods known in the art. For example a sulfonate group can be reduced to a hydrogen by a reductive reaction (such as the use of lithium aluminum hydride) or exchanged to an oxygen function by a displacement reaction of sodium benzoate. For preparation of oligomers of formula I when X is NHacyl, the acyl group chosen can be introduced during the capping step of conventional solid phase oligomer synthesis.

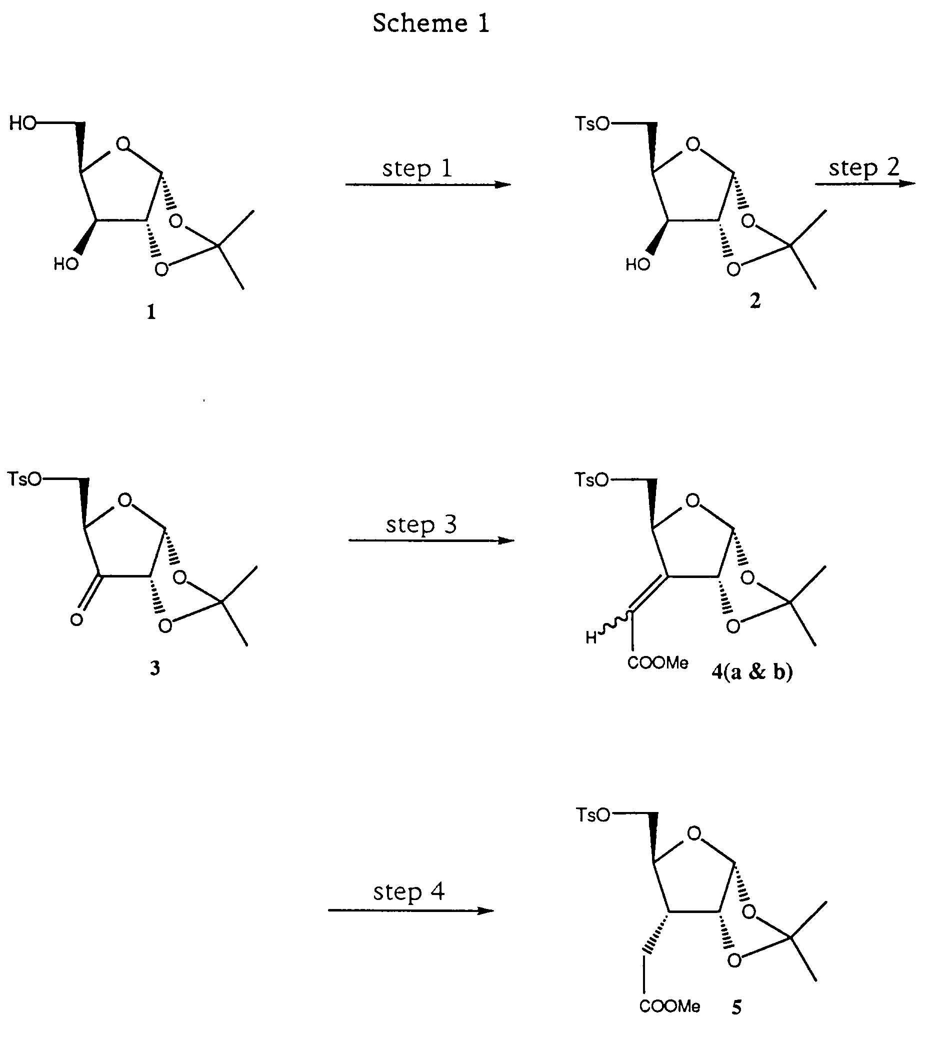

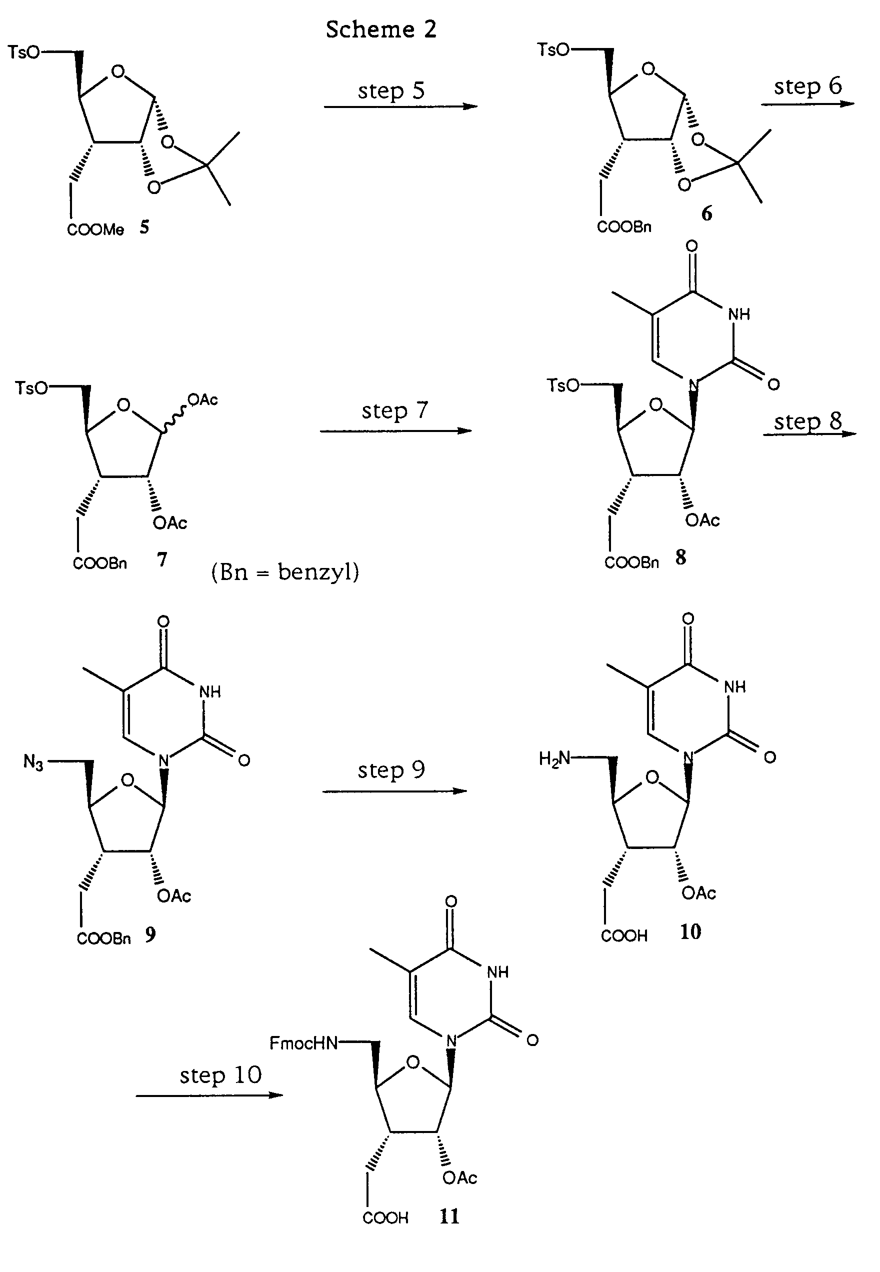

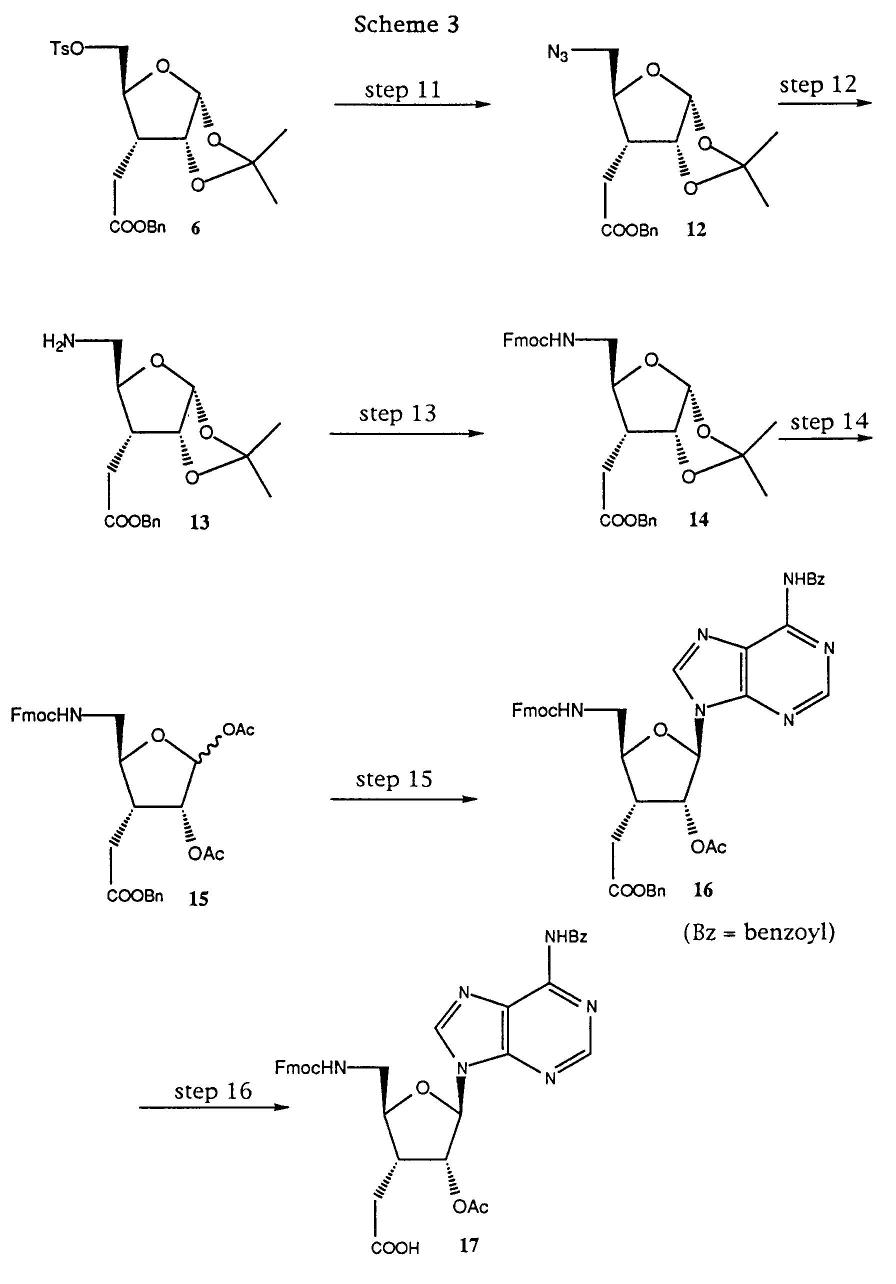

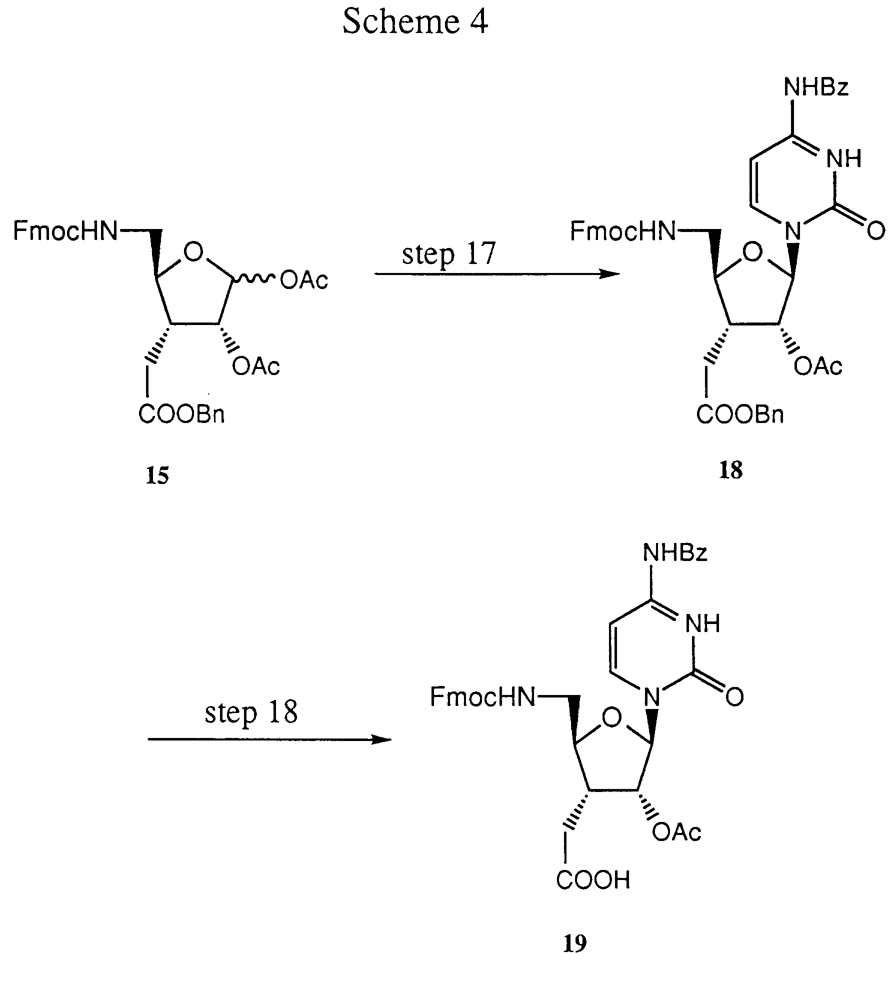

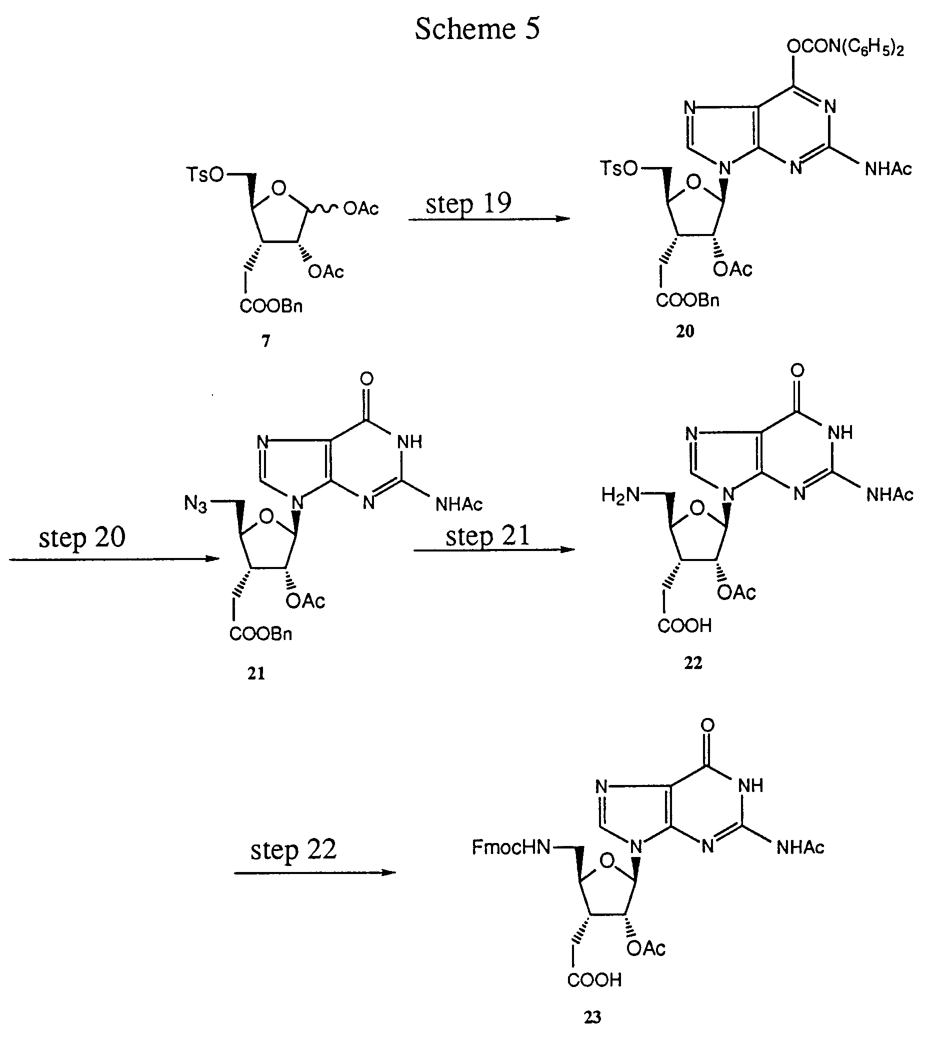

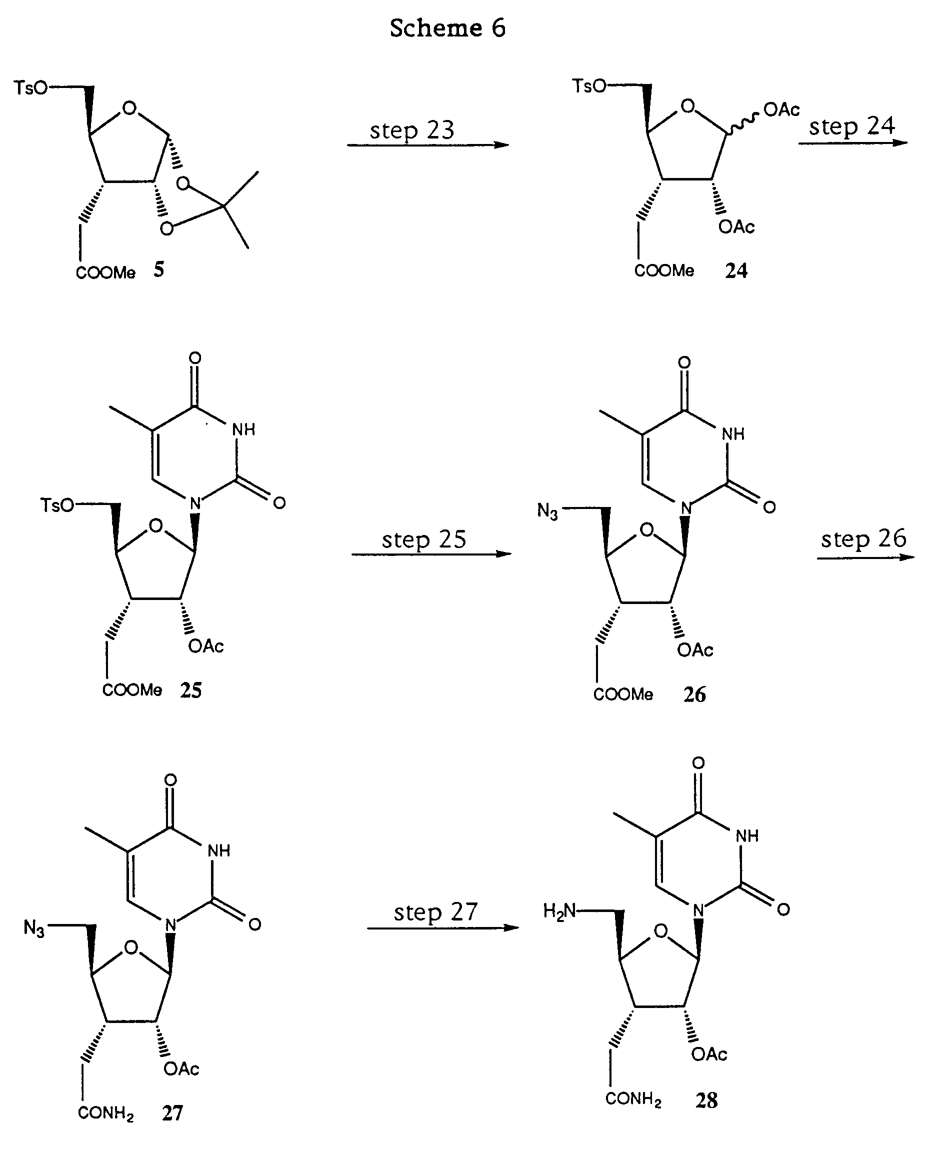

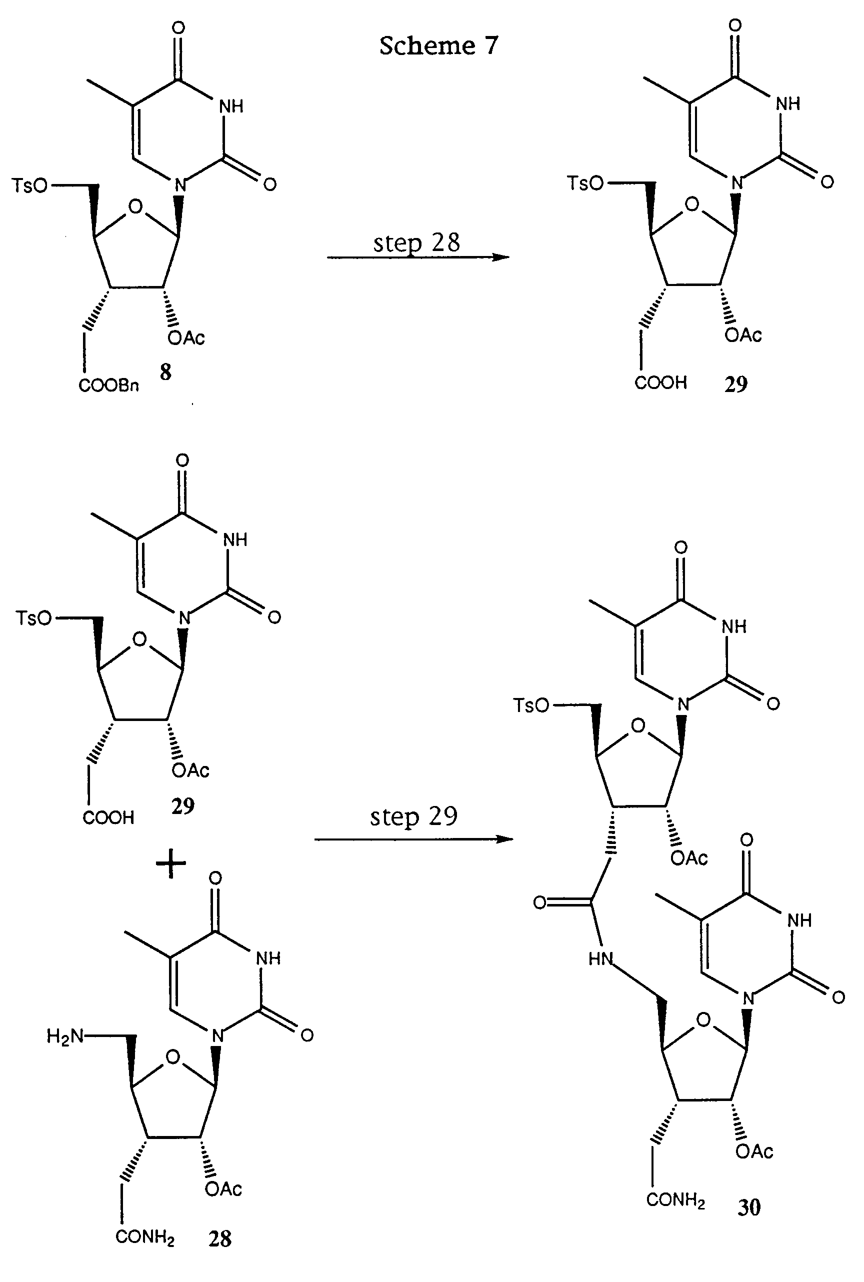

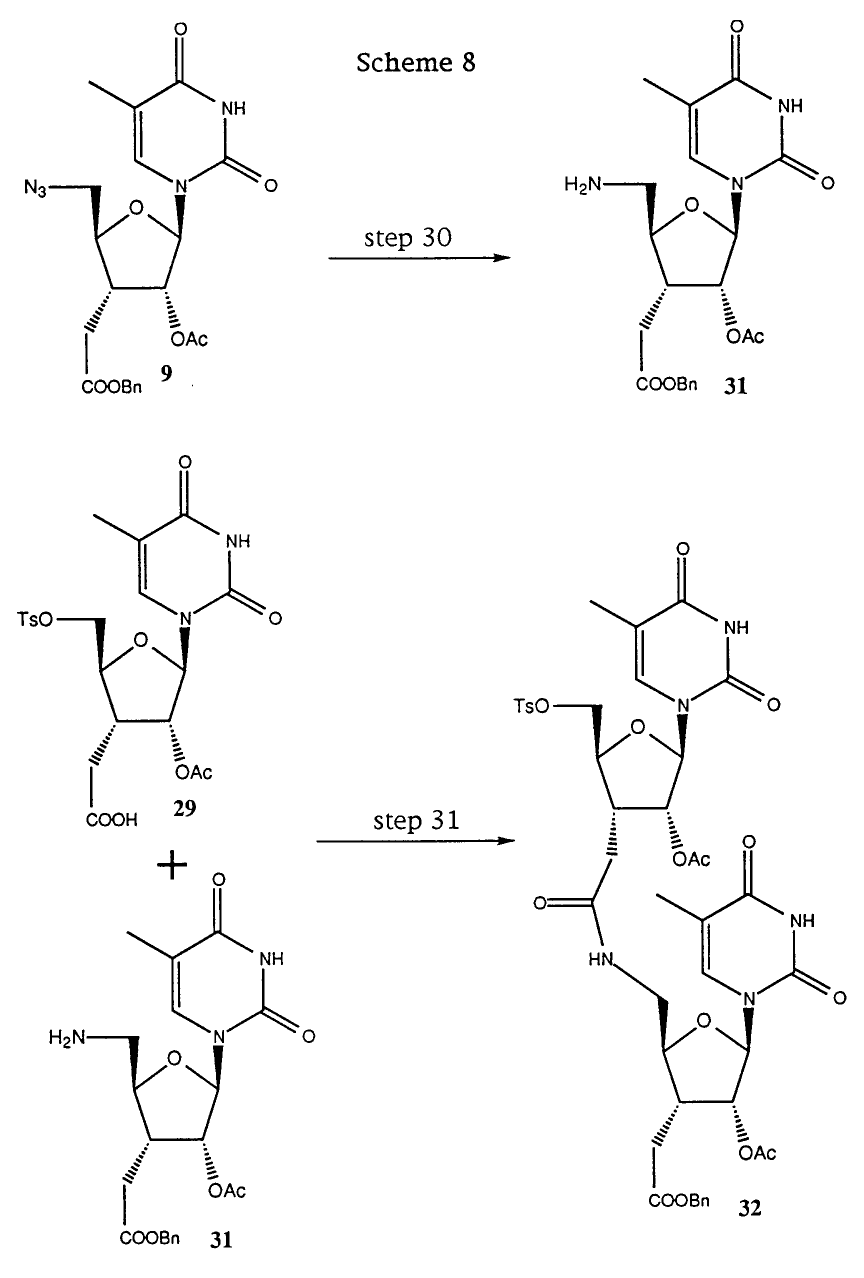

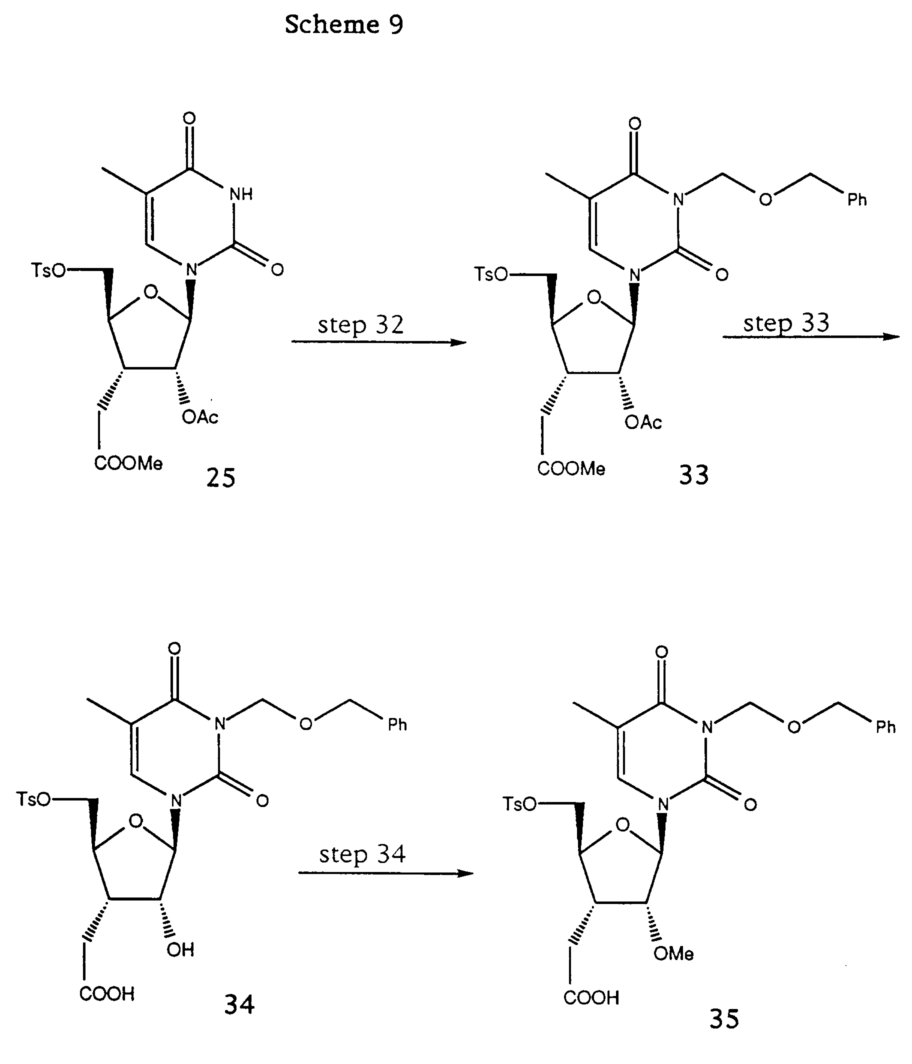

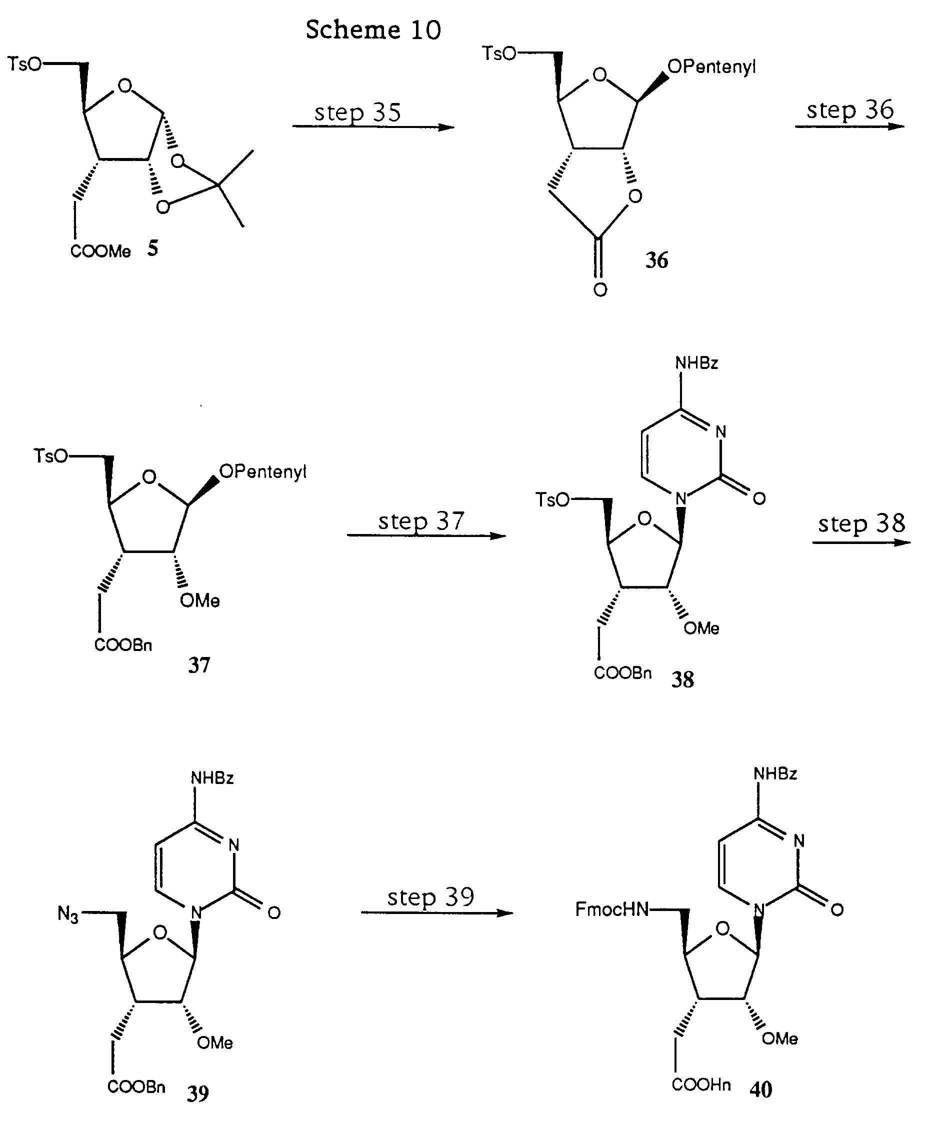

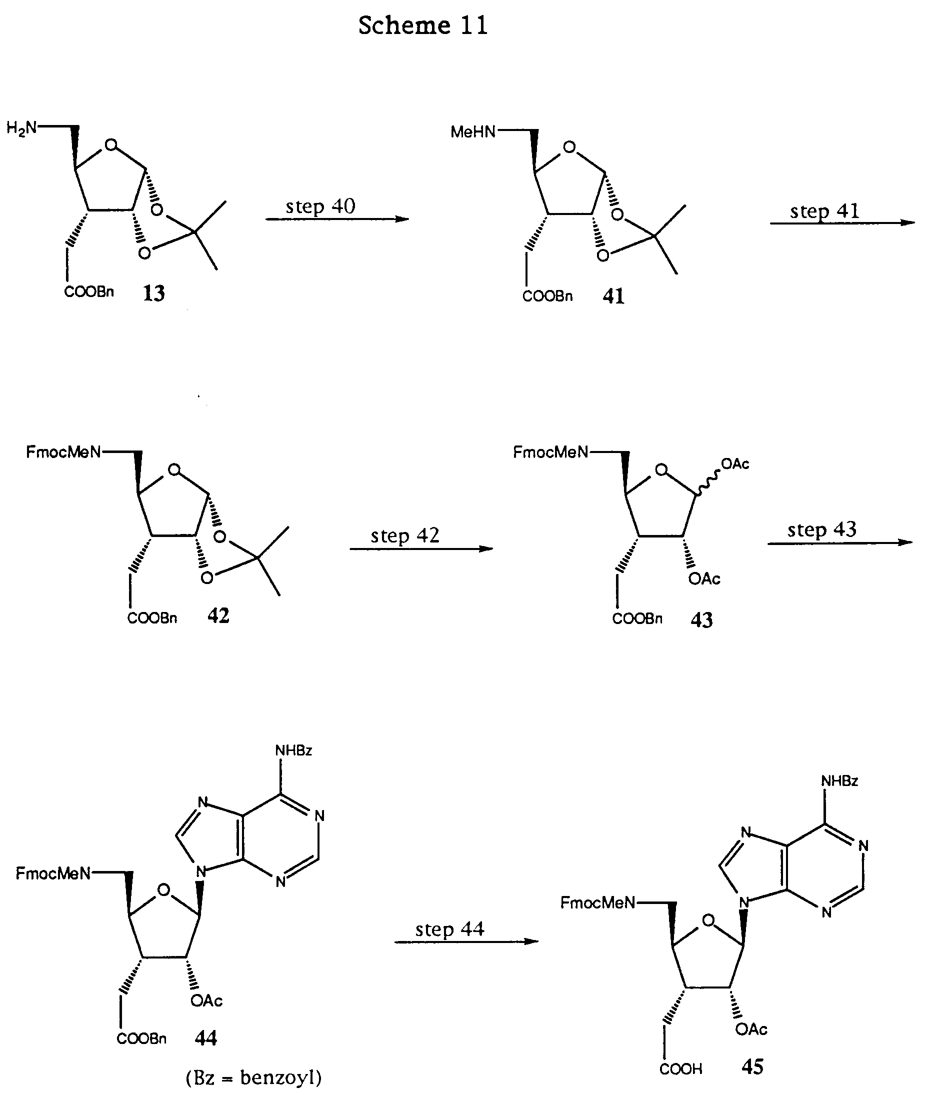

### EXPERIMENTAL

### Example 1

### Synthesis of Thymine Building Block, 11

### Step 1: Synthesis of compound 2

A solution of 40.0 g of 1,2-isopropylidene-a-D-xylofuranose **1** (pfanstiehl) in 125 ml of CH₂Cl₂ was treated with 40 ml of dry pyridine, and then cooled to -5 °C. It was then treated with a solution of 44.2 g of tosyl chloride in 140 ml of CH₂Cl₂ over a period of 30 minutes. After stirring overnight during which time the reaction mixture was allowed to warm to room temperature, the reaction mixture was diluted with 500 ml of CH₂Cl₂, and washed with 800 ml water, and finally with 400 ml of brine. The organic layer was dried over Na₂SO₄, filtered, concentrated and then further dried under high vacuum overnight to yield 75.0 g of the crude product as an off-white solid. The crude product was crystallized using CH₂Cl₂/Hexane to give 56.7 g of the pure tosylate **2**. Confirmed by ¹H-NMR(ppm) 2.44 (3H, tosyl-CH₃), 5.89 (1H, Anomeric-H-1), 7.34 & 7.80 (4H, tosylphenyl).

### Step 2: Synthesis of compound 3

A solution of 7.24 g of the tosylate **2** and 130 ml of DMSO was treated with 84 ml of acetic anhydride and the reaction mixture was then stirred overnight at room temperature. During the work up it was diluted with 250 ml of EtOAc and 100 ml of water and stirred for half hour and separated the organic layer. The aqueous layer was washed with 250 ml of EtOAc. The organic layers were combined and washed with 800 ml of water, followed by 400 ml of sat. NaHCO₃. The organic layer was separated and washed finally with 200 ml of brine. It was then dried over Na₂SO₄ and concentrated to give the crude product. Purification was carried out over a silica gel column using Hexane/EtOAc in the ratio 1.1:1 as eluant to give 5.54 g of ketone **3** as a very pale yellow oil. Confirmed by ¹H-NMR(ppm) 2.44 (3H, tosyl-CH₃), 6.15 (1H, Anomeric-H-1); IR(cm⁻¹) 1783 (ketone stretch); (+)EI (MW) 342.

### Step 3: Synthesis of compound 4(a & b)

A solution of 4.01 g of the ketone **3** and 80 ml of CH₃CN was treated with 7.83 g of methyl(triphenylphosphoranylidene) acetate. This pale yellow reaction mixture was heated to reflux for 3.5 h after which it was cooled to room temperature and concentrated to dryness. The residue obtained was redissolved in minimum amount of CH₂Cl₂ and was loaded on a silica gel column and eluted using Hexane/EtOAc (1.9:1) as eluant to afford a combined yield of 2.74 g of the alkenes **4a** and **4b**. Confirmed by ¹H-NMR(ppm) 2.44 (3H, tosyl-CH₃), 5.8-5.9 (1H, Alkene-H); IR(cm⁻¹) 1725 (ester stretch); (+)EI (MW) 398.

### Step 4: Synthesis of compound 5

A solution of 1.65g of the alkene **4** in 20 ml of EtOAc was stirred with 10 % Pd/C under hydrogen atmosphere for 7 h under S.T.P. conditions. The solution was then filtered through a celite pad. Concentration of the filtrate afforded the crude product as a white solid. The crude product was crystallized using MeOH/Et₂O to give 1.18 g of the saturated ester **5**. Confirmed by ¹H-NMR(ppm) 2.45 (3H, tosyl-CH₃), 3.71 (3H, COOCH₃), 5.71 (1H, Anomeric-H-1); IR(cm⁻¹) 1734 (ester stretch); (+)FAB (M+1) 401.

### Step 5: Synthesis of compound 6

A solution of 7.3 g of the methyl ester **5** in 70 ml of THF was treated with 40 ml of 0.5 N NaOH at room temperature and stirred for 3 h. After the reaction was completed it was concentrated to dryness and then the residue was taken in 70 ml of DMF at 0 °C. This solution was then treated with 5.2 g of NaHCO₃ and 3.3 ml of benzyl bromide. The reaction was allowed to stir overnight during which it warmed up to room temperature.

During the work up it was concentrated to dryness and the residue was partitioned between 200 ml of CH₂Cl₂ and 50 ml of H₂O. The organic layer was separated and washed with brine after which it was dried over Na₂SO₄ and concentrated to give the crude product. This was then washed with 2 x 100 ml hexane, decanted the hexane layers and dried the solid under high vacuum to yield 7 g of the pure benzyl ester **6** as a white solid. Confirmed by ¹H-NMR(ppm) 5.70 (1H, Anomeric-H-1), 5.14 & 5.16 (2H, PhCH₂); (+)FAB (M+1) 477; IR(cm⁻¹) 1733 (ester stretch).

### Step 6: Synthesis of compound 7

A solution of 8.48 g of the benzyl ester **6** in 105 ml of CH₂Cl₂ at -78 °C treated with 5 ml of bromodimethyl borane and stirred for 2 h. The cooling bath was removed and the reaction was allowed to stir for an additional 3 h. The reaction mixture was slowly poured into a well stirred solution(150 ml) of sat. NaHCO₃/TNF(4:1) over a period of 10 min. The solution was then stirred for another 15 min. It was then diluted with 250 ml CH₂Cl₂ and 10 ml H₂O. The organic layer was separated and washed with 100 ml brine, dried over Na₂SO₄ and concentrated to give 7.8 g of the crude dihydroxy intermediate.

The crude product was dissolved in 18 ml of dry acetic anhydride and cooled to 0 °C. It was then treated with 8.5 ml of pyridine and stirred overnight during which it warmed up to room temperature. During the work up it was quenched with 10 ml of H₂O at 0 °C and stirred for 15 min. The reaction mixture was then concentrated to dryness under high vacuum at 50 °C. The residue was dissolved in 300 ml of EtOAc and washed successively with 125 ml of H₂O, 125 ml of brine. The EtOAc layer was then dried over Na₂SO₄ and concentrated to give 8.6 g of the diacetate 7 as a foamy solid. Confirmed by ¹H-NMR(ppm) 6.22 (1H, Anomeric-H-1), 2.01 & 2.04 (6H, OCOCH₃); (+)FAB MW 520; IR(cm⁻¹) 1746 (ester stretch).

### Step 7: Synthesis of compound 8

A suspension of 8.6 g of diacetate **7** and 7.21 g of N,O-bis(trimethylsilyl)-thymine in 105 ml of dichloroethane was treated with 2.6 ml 0f freshly distilled tin(IV) chloride and the reaction mixture was stirred at room temperature for 2 h after which it was refluxed gently at 90 °C for 1 h. The reaction mixture was then cooled to room temperature and diluted with 25 ml of CH₂Cl₂ and quenched with 10 ml of H₂O.
After stirring for 30 min. it was diluted further with 300 ml of CH₂Cl₂ and 150 ml of H2O. The organic layer was then separated and washed with 150 ml of brine solution and dried over Na₂SO₄, concentrated to give 8.8 g of the nucleoside **8** as a white foamy solid.
Confirmed by ¹H-NMR(ppm) 5.80 (1H, Anomeric H-1), 7.92 (s, 1H, CONHCO); (+)FAB (M+1) 587; IR(cm-1) 1694 (Amide stretch).

### Step 8 : Synthesis of compound 9

A solution of 8.8 g of the nucleoside **8** in 80 ml of DMF was stirred vigorously with 4.94 g of NaN₃ and the reaction mixture was warmed to 75 °C. The reaction mixture was stirred at this temperature for 6 h. It was then cooled to room temperature and was concentrated to dryness on a rotary evaporator at 50 °C. The residue was partitioned between 300 ml of EtOAc and 200 ml of H₂O
The organic layer was separated and washed with 100 ml of brine solution, dried over Na₂SO₄, concentrated, and then chromatographed with 19:1 CH2Cl2/MeOH to give 5.7 g of the azide **9**. Confirmed by ¹H-NMR(ppm) 5.77 (1H, Anomeric H-1); (+)FAB (M+1) 458; IR(cm⁻¹) 2107 (N₃).

### Step 9 : Synthesis of compound 10

A mixture of 4.14g of the azide **9** in 50 ml 0f MeOH and 1.06g of 10 % Pd/C was stirred under H2 atmosphere at STP conditions. The reaction mixture was stirred for 20 h after which it was filtered over a celite pad. The filter cake was washed with 25 ml of MeOH and the combined filtrates were concentrated to give 3.08 g of the amino acid **10** which was used as such for the next step. Confirmed by 1H-NMR(ppm) 5.77 (1H, Anomeric H-1); (+)FAB (M+1) 342; IR(cm⁻¹) 3000 (COOH stretch).

### Step 10: Synthesis of compound 11

A solution of the amino acid **10** and 3.39 g of N-(9-Fluorenylmethoxycarbonyloxy)-succinmide in 50 ml of 1,4-dioxane/H₂O(1:1) was cooled to 0 °C. It was then treated with 1.95 g of NaHCO₃ and stirred for 4 h. The cooling bath was removed and it was continued to stir at room temperature for an additional 4 h. During the work up the reaction mixture was concentrated to dryness on a rotary evaporator at 50 °C. The residue was taken into 200 ml of H₂O and 75 ml of EtOAc. The aqueous layer was separated and acidified with 1.0 N HCl to pH 4, and then lyophilized. The crude product was purified on a reverse phase silica gel column with H₂O/CH₃CN in the ratio 1.86:1 as the eluant to give 2 g of the pure compound **11** as a white solid. Confirmed by ¹H-NMR(ppm) 5.64 (1H, Anomeric-H-1), 11.4 (1H, FmocHN); (+)FAB (M+1) 564; IR(cm⁻¹) 1696 (amide stretch), 3401 (COOH stretch).

### Example 2

### Synthesis of Adenine Building Block, 17

### Step 11: Synthesis of compound 12

A solution of 712 mg of the tosylate **6** in 6.6 ml of DMF was stirred vigorously with 512 mg of sodium azide and the reaction mixture was warmed to 65 °C. The reaction mixture was stirred at this temperature for 5 h. It was then cooled to room temperature and was concentrated to dryness on a rotary evaporator at 45 °C. The residue was partitioned between 150 ml of EtOAc and 75 ml of H₂O. The organic layer was separated and washed with 40 ml of brine solution, dried over Na₂SO₄, concentrated, and then chromatographed with 2:1 hexane/EtOAc to give 467 mg of the azide **12**. Confirmed by ¹H-NMR(ppm) 5.82 (1H, Anomeric H-1), 5.15 (2H, PhCH₂); IR(cm⁻¹) 2100 (N₃)

### Step 12: Synthesis of compound 13

A solution of 130 mg of the azide **12** in 5 ml of EtOAc was stirred with 58 mg of Lindlaar's catalyst under H₂ atmosphere at STP conditions. The reaction mixture was stirred for 6 h after which it was filtered over a celite pad. The filter cake was washed with 25 ml of EtOAc and the combined filtrates were concentrated to give 100 mg of the amine **13** which was used as such for the next step. Confirmed by ¹H-NMR(ppm) 5.77 (1H, Anomeric H-1), 5.15 (2H, PhCH₂); IR(cm⁻¹) No azide stretch.

### Step 13 : Synthesis of compound 14

A solution of 100 mg of amine **13** in 2 ml of acetonitrile was sequentially treated with 0.07 ml of triethylamine and 88 mg of FMOC-Cl and the reaction mixture was stirred at room temperature for 3 h. The reaction mixture was then concentrated to dryness. The residue obtained was redissolved in 2 ml of CH₂Cl₂ and loaded on a silica gel column and eluted using 2:1 Hexane/EtOAc to give 95 mg of pure compound **14**. Confirmed by ¹H-NMR(ppm) 5.80 (1H, Anomeric H-1), 5.15 (2H, PhCH₂), 4.20-4.40 (3H, Fmoc-CH₂CH); IR(cm⁻¹) 3451 (NH stretch), 1725 (COOBn stretch); (+)FAB (M+1) 544.

### Step 14: Synthesis of compound 15

A solution of the compound **14** in 55 ml CH₂Cl₂ at -78 °C was treated with 2.9 ml of bromodimethyl borane and stirred for 1.5 h. The reaction mixture was warmed to 0 °C and allowed to stir for an additional hour. During the work up it was quenched with 10 ml of saturated NaHCO₃ and stirred for 30 min. The reaction mixture was then diluted with 300 ml of EtOAc and 75 ml of H₂O. The organic layer was separated and washed with 100 ml of saturated NaHCO₃ 100 ml of brine, dried over Na₂SO₄ and concentrated to give the crude dihydroxy intermediate.

The crude product was dissolved in 5.4 ml of dry acetic anhydride and cooled to 0 °C. It was then treated with 12.0 ml of dry pyridine and stirred overnight during which it was warmed to room temperature. During work up it was quenched with 10 ml of H₂O at 0 °C and stirred for 15 min. The reaction mixture was then concentrated to dryness under high vacuum at 50 °C. The residue was dissolved in 300 ml EtOAc and washed successively with 125 ml of H₂O, 125 ml of brine. The EtOAc layer was dried over Na₂SO₄ and concentrated to give the crude product which was chromatographed with 9:5 Hexane/EtOAc to give 3.85 g of the diacetate **15** as a foamy white solid. Confirmed by ¹H-NMR(ppm) 4.23-4.42 (3H, Fmoc-CH₂CH), 6.36 (1H, Anomeric H-1); IR(cm⁻¹) 3400 (broad NH stretch), 1740 (COOBn stretch); (+)FAB MW 587.

### Step 15 : Synthesis of compound 16

A suspension of 1.51 g of diacetate **15** and 9.5 g of N-6,7-bis(trimethylsilyl)-N-6-benzoyl adenine in 20 ml of toluene was treated with 1.0 ml of TMSOTf and the reaction mixture was stirred at room temperature overnight. The reaction mixture was then concentrated to dryness under high vacuum at 45 °C. The residue was partitioned between 250 ml of EtOAc and 125 ml of H₂O. The organic layer was separated and washed with 100 ml of saturated NaHCO₃, 100 ml of brine, dried over Na₂SO₄ concentrated and chromatographed with 65:1 CH₂Cl₂/MeOH to give 920 mg of pure **16** as a white solid. Confirmed by ¹H-NMR(ppm) 2.02 (1H, OCOCH₃), 4.20-4.40 (3H, Fmoc-CH₂CH), 5.95(1H, Anomeric H-1), 6.92 (1H, FmocHN), 8.06 (1H, Adenine-C-2H), 8.71 (1H, Adenine-C-8H), 8.97 (1H, NHCOPh); IR(cm⁻¹) 3400 (broad NH stretch), 1737 (COOBn stretch), 1714 (NHCOO-urethane stretch); (+)FAB (M+1) 767.

### Step 16: Synthesis of compound 17

A mixture of 760 mg of the nucleoside **16** in 15 ml of MeOH and 5 drops of HCOOH was stirred with 310 mg of 10 % Pd/C under H₂ atmosphere at STP conditions. The reaction mixture was stirred for 18 h after which it was filtered over a celite pad. The filter cake was washed with 25 ml of MeOH and the combined filtrates were concentrated to give the crude product which was purified on a reverse phase silica gel column with H₂O/CH₃CN in the ratio 1.5:1 as the eluant to give 432 mg of pure compound **17** as a white solid. Confirmed by ¹H-NMR(ppm) 2.16 (1H, OCOCH₃), 4.28-4.45 (3H, Fmoc-CH₂CH), 6.03(1H, Anomeric H-1), 7.20 (1H, FmocHN), 8.16 (1H, Adenine-C-2H), 8.75 (1H, Adenine-C-8H), 9.42 (1H, NHCOPh); IR(cm⁻¹) 3400 (broad NH stretch), 1709 & 1720 (broad stretch for acid and ester); LR-EI (M+1) 677.

### Example 3

### Synthesis of Cytosine Building Block, 19

### Step 17: Synthesis of Compound 18

A solution of 91 mg of diacetate **15** and 76 mg of Trimethylsilyl-N-6-benzoyl cytosine in 2 mL of Toluene was treated with 0.07 mL of TMSOTf and the reaction mixture was stirred at room temperature overnight. The reaction was diluted with 50 mL of CH₂Cl₂, washed with 25 mL of brine followed by 25 mL of saturated NaHCO₃, dried over Na₂SO₄ and concentrated to dryness. The residue was recrystallized from methanol to give 69 mg of pure **18** as a white solid. Confirmed by 1H-NMR (ppm) 2.02 (3H, OCOCH₃), 4.25-4.50 (3H, Fmoc-CH₂CH), 5.11 (2H, CH₂Ph), 5.63 (1H, C-1'-H), 8.66 (1H, NHCOPh); (+) FAB MW 742.

### Step 18: Synthesis of Compound 19

A solution of 0.65 g of Benzyl ester **18** was dissolved in 65 mL of Dioxane and 0.1% Formic acid and stirred with 285 mg of 10% Pd/C under H₂ atmosphere at STP conditions. The reaction was stirred for 64 hours after which it was filtered over a Celite pad. The filter cake was washed with 200 mL CH₂Cl₂ and the combined filtrates were concentrated to give the crude product which was chromatographed with CH₂Cl₂/MeOH to give 356 mg of pure **19** as a white solid. Confirmed by ¹H-NMR (ppm) 2.10 (3H, COCH₃), 4.20-4.40 (3H, Fmoc-CH₂CH), 5.49 (1H, C-2 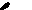 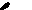 -H), 5.68 (1H, C-1 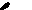 -H), 8.23 (1H, Cytosine-C-6H): UV (MeOH) max 261 nm, E=37,600; max 288 nm E=11,900; IR (cm⁻¹) 1706 (Acid stretch), 1671 (Amide stretch); (+) FAB MW 652; CHN calc for C₃₅H₃₂N₄O₉·1H₂O, C=62.68, H=5.11, N=8.35; found C=62.29, H=4.92, N=8.20.

### Example 4

### Synthesis of Guanine Building Block, 23

### Step 19: Synthesis of Compound 20

A solution of 0.26 g of Tosyldiacetate **7** and 0.39 g of Trimethylsilyl-N-2-acetyl-6-O-diphenylcarbarnoyl guanine in 4 mL Toluene was treated with 0.12 mL of TMSOTf and stirred at 50° for 2 h. The mixture was cooled to room temperature and then treated with 0.9 mL of triethylamine and evaporated to an oil. This crude product was chromatographed with CH₂Cl₂/Ethyl Acetate 1:1 to give 216 mg of pure nucleoside **20**. Confirmed by ¹H-NMR (ppm) 2.00 (3H, COCH₃), 2.29 (3H, COCH₃), 2.37 (3H, PhCH₃), 5.11 (2H, OCH₂Ph), 5.62 (1H, C-2 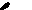 -H), 5.81 (1H, C-1 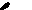 -H), 7.89 (1H, purine C-8-H), 8.03 (1H, NHOCH₃); (+) FAB MW 848; UV (MeOH) max 226 nm, E=38,400, max 278nm, E=11,200; IR(cm⁻ ¹)3428-3396 (wide amine stretch), 1740 (ester stretch) 1695 (Acetate and Amide stretch); CHN calc for C₄₃H₄₀N₆O₁₁S, C=60.84, H=4.75, N=9.90, found C=60.89, H=4.65, N=9.83.

### Step 20: Synthesis of Compound 21

A solution of 0.85 g of the Tosylnucleoside **20** and 2.3 g of Lithium azide and 25 mL DMF was stirred at 60° for 5h. The reaction mixture was evaporated and then partitioned between CH₂Cl₂ and brine. The organic layer was dried over Na₂SO₄, evaporated and chromatographed with CH₂Cl₂/MeOH 25:1 to give 265 mg of the azide **21**. Confirmed by ¹H-NMR (ppm) 2.08 (3H, COCH₃), 2.27 (3H, COCH₃), 5.14 (2H, OCH₂Ph), 5.80 (1H, C-1 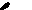 -H), 5.91 (1H, C-2 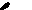 -H), 7.79 (1H, purine-C-8-H), 8.88 (1H, NH); (+) FAB MW 524; UV (pH1) max 260 nm, E=17,400, (pH7) max 258 nm, E=16,850, (pH12) max 264, E=13,900; IR (cm⁻¹) 2102 (Azide stretch), 1741 (Ester stretch), 1678 (Amide stretch); CHN calc for C₂₃H₂₄N₈O₇, C=52.67, H-4.61, N=21.36, found C=52.11, H=4.47, N=20.95.

### Step 21: Synthesis of Compound 22

A solution of 0.28 g of azide **21** in 15 mL MeOH was stirred with 114 mg of 10% Pd/C under H₂ atmosphere at STP conditions. The reaction was stirred for 5 hours and then filtered over a Celite pad. The filter cake was washed with methanol and then with water. The filtrates were evaporated to give 192 mg of crude amino acid **22**.

### Step 22: Synthesis of Compound 23

A solution of 192 mg of amino acid **22** in 50 mL of (1:1) CH₃CN/THF was treated with 0.21 mL of Triethylamine and 199 mg of Fmoc-hydroxy-succinimide first at 4° then at room temperature for 1 h. The reaction mixture was evaporated to dryness, dissolved in CH₂Cl₂/MeOH (5:2) to give 139 mg of pure compound **23**. Confirmed by ¹H-NMR(ppm) 2.08 (3H, COCH₃), 2.15 (3H, COCH₃), 4.19 (3H, Fmoc CHCH₂), 5.61 (1H, C-2 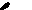 -H), 5.87 (1H, C-1 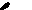 -H); (+) FAB MW 630; UV (MeOH) max 261 nm, E=27,600, max 288 nm, E=12,500; IR (cm⁻¹) 2880-3500 (Broad OH and NH stretch).

### Example 5

### Synthesis of Dimer, 30

### Step 23 : Synthesis of compound 24

A solution of 1.04 g of the methyl ester **5** in 12 ml of CH₂Cl₂ at -78 °C was treated with 1.1 ml of bromodimethyl borane and stirred for 2 h. The cooling bath was removed and the reaction was allowed to stir for an additional hour. The reaction mixture was slowly poured into a well stirred solution (75 ml) of sat. NaHCO₃/TNF(2:1) over a period of 10 min. The solution was then stirred for another 20 min. It was then diluted with 150 ml of CH₂Cl₂ and 100 ml of H₂O. The organic layer was separated and washed with 100 ml of brine, dried over Na₂SO₄ and concentrated to give 7.8 g of the crude dihydroxy intermediate.

The crude product was dissolved in 10.0 ml of dry acetic anhydride and cooled to 0 °C. It was then treated with 5.0 ml of dry pyridine and stirred overnight during which it was warmed to room temperature. During work up it was quenched with 10 ml of H₂O at 0 °C and stirred for 15 min. The reaction mixture was then concentrated to dryness under high vacuum at 50 °C. The residue was dissolved in 150 ml of EtOAc and washed successively with 50 ml of H₂O, 125 of ml brine. The EtOAc layer was then dried over Na₂SO₄ and concentrated to give 1.07 g of the diacetate **24** as a foamy solid. Confirmed by ¹H-NMR(ppm) 6.03 & 6.20 (1H, Anomeric-H-1-a & b), 3.70 (s, 3H, COOCH₃), 2.06 & 2.09 (s, 6H, OCOCH₃); IR(cm⁻¹) 1745(ester stretch).

### Step 24: Synthesis of compound 25

A suspension of 2.85 g of diacetate **24** and 2.37 g of N,O-bis(trimethylsilyl)-thymine in 33 ml of dichloroethane was treated with 0.75 ml of freshly distilled tin(IV) chloride and the reaction mixture was stirred at room temperature for 2 h after which it was refluxed for 1 h. The reaction mixture was then cooled to room temperature and diluted with 250 ml of CH₂Cl₂ and quenched with 10 ml of H₂O.

After stirring for 30 min. it was diluted further with 50 ml of CH₂Cl₂ and 100 ml of H₂O. The organic layer was then separated and washed with 150 ml of brine solution and dried over Na₂SO₄, concentrated to give the crude product which was chromatographed using CH₂Cl₂/MeOH (30:1) as eluant to give 3.15 g of the nucleoside **25** as a white foamy solid. Confirmed by ¹H-NMR(ppm) 5.69 (1H, Anomeric H-1), 5.40 (1H, H-2), 3.64 (3H, COOCH₃), 2.06 (3H, OCOCH₃), 1.89 (3H, thymine-CH₃); (+)FAB (M+1) 511; IR(cm⁻¹) 1693 (lactam stretch), 1739 (ester stretch).

### Step 25 : Synthesis of compound 26

A solution of 263 mg of the nucleoside **25** in 3.0 ml of DMF was stirred vigorously with 234 mg of sodium azide and the reaction mixture was warmed to 75 °C. The reaction mixture was stirred at this temperature for 3 h. It was then cooled to room temperature and was concentrated to dryness on a rotary evaporator at 50 °C. The residue was partitioned between 100 ml of EtOAc and 50 ml of H₂O. The organic layer was separated and washed with 25 ml of brine solution, dried over Na₂SO₄, concentrated, and then chromatographed with 30:1 CH₂Cl₂/MeOH to give 190 mg of the azide **26**. Confirmed by ¹H-NMR(ppm) 5.73 (1H, Anomeric H-1), 5.48 (1H, H-2), 3.70 (3H COOCH₃), 1.90 (3H, thymine-CH₃); (+)FAB (M+1) 382; IR(cm⁻¹) 2107 (N₃), 1739 (ester stretch), 1694 (lactam stretch).

### Step 26: Synthesis of compound 27

A solution of 98 mg of the azide **26** in 3 ml of methanol was cooled to 0 °C and anhydrous ammonia gas was bubbled into this solution for approximately 5 minutes. The reaction flask was sealed and the resulting solution was stirred for about 12 h after which the solvent was removed and the crude hydroxy amide was chromatographed using 9:1 CH₂Cl₂/MeOH as the eluant to give 68 mg of the pure product.

This compound was then dissolved in 1.0 ml of pyridine and treated with 0.1 ml of acetic anhydride at 0 °C. After stirring for 12 h the reaction was quenched with few drops of water and was concentrated to dryness. The crude acetate obtained was chromatographed using 12:1 CH₂Cl₂/MeOH as the eluant to give 63 mg of pure **27**. Confirmed by ¹H-NMR(ppm) 5.77 (1H, Anomeric H-1), 5.44 (1H, H-2), 2.10 (3H, OCOCH₃), 1.90 (3H, thymine-CH₃); (+)FAB (M+1) 367; IR(cm⁻¹) 3408 (broad NH stretch), 2122 (azide stretch), 1750 (ester stretch), 1719 (lactam stretch), 1665 (amide stretch).

### Step 27 : Synthesis of compound 28

A mixture of 60 mg of the azide **27** in 3.5 ml of MeOH and 20 mg of 10 % Pd/C was stirred under H₂ atmosphere at STP conditions. The reaction mixture was stirred for 30 min. after which it was filtered over a celite pad. The filter cake was washed with 25 ml of MeOH and the combined filtrates were concentrated to give the crude product which was chromatographed using 9:1:0.1 CH₂Cl₂/MeOH/isopropyl amine to give 39 mg of the pure amine **28**. Confirmed by ¹H-NMR(ppm) 5.66 (1H, Anomeric H-1), 5.41 (1H, H-2), 2.10 (3H, OCOCH₃), 1.89 (3H, thymine-CH₃); (+)FAB (M+1) 341; IR(cm⁻¹) 3400 (broad NH stretch), 1740 (ester stretch), 1692 ( broad lactam and amide stretch).

### Step 28 : Synthesis of compound 29

A solution of 32 mg of the benzyl ester **8** in 2.0 ml of MeOH was treated with 0.12 ml of 1,4-cyclohexadiene and stirred vigorously with 15 mg of 10 % Pd/C and the reaction mixture was stirred at room temperature for 16 h. It was then concentrated to dryness on a rotary evaporator to give 28 mg of the carboxylic acid **29**. Confirmed by ¹H-NMR(ppm) 5.70 (1H, Anomeric H-1), 5.00 (1H, H-2), 2.43 (3H, tosyl-CH₃), 2.07 (3H, OCOCH₃); (+)FAB (M+1) 496; IR(cm⁻¹) 1747 (ester stretch), 1695 & 1700 (acid & lactam stretch).

### Step 29 : Synthesis of compound 30

A solution of 19.5 mg of the acid **29** and 14.5 mg of the amine **28** in 1.5 ml of DMF was treated with 0.027 ml of diisopropylethyl amine followed by 24 mg of HBTU at room temperature. The reaction mixture was stirred for 16 h after which it was concentrated to dryness and the residue obtained was dissolved in minimum amount of MeOH and loaded on a silica gel column and eluted using 7:1:1:1 EtOAc/MeCN/MeOH/H₂O to give 25 mg of pure dimer **30**. Confirmed by ¹H-NMR(ppm) 5.59 & 5.68 (2H, Anomeric H-1's), 5.00 & 5.04 (2H, H-2's), 2.42 & 2.81 (6H, two-tosyl CH₃'s), 2.08 & 2.09(6H, two-OCOCH₃'s), 1.87 & 1.89 (6H, two-thymine-CH₃'s); (+)FAB (M+1) 819; IR(cm⁻¹) 1690 to 1700 (broad stretch-amide, ester and lactam).

### Example 6

### Synthesis of Dimer Building Block, 32

### Step 30: Synthesis of compound 31

A solution of 350 mg of the azide **9** in 7 ml of EtOAc was stirred with 600 mg of Lindlaar's catalyst under H₂ atmosphere at STP conditions. The reaction mixture was stirred for 6 h after which it was transferred on to a paar hydrogenator and shaken for 6 h at 50 psi H₂ pressure. The reaction mixture was then filtered over a celite pad. The filter cake was washed with 50 ml of EtOAc and the combined filtrates were concentrated to give the crude product which was chromatographed using 7:1:1:1 EtOAc/MeCN/MeOH/H₂O to give 165 mg of the pure amine **31**. Confirmed by ¹H-NMR(ppm) 5.45 & 5.63 (2H, Anomeric H-1 & H-2), 5.12-5.16 (2H, PhCH₂); (+)FAB (M+1) 432; IR(cm⁻¹) 3393 (NH₂ stretch), 1738 (ester stretch), 1692 (lactam stretch)

### Step 31: Synthesis of compound 32

A solution of 179 mg of the acid **29** and 160 mg of the amine **31** in 3.0 ml of DMF was treated with 0.18 ml of diisopropylethyl amine followed by 146 mg of HBTU at room temperature. The reaction mixture was stirred for 3 h after which it was concentrated to dryness and the residue obtained was dissolved in minimum amount of MeOH and loaded on a silica gel column and eluted using 7:1:1:1 EtOAc/MeCN/MeOH/H₂O to give 200 mg of pure dimer **32**. Confirmed by ¹H-NMR(ppm) 5.45 (2H, Anomeric H-1's), 5.82 (2H, H-2's), 2.43 & 2.12 (12H, two-tosyl CH₃'s & two-OCOCH₃'s), 1.90 & 1.93 (6H, two-thymine-CH₃'s); (+)FAB (M+1) 910; IR(cm⁻¹) 1738 (ester stretch) 1693 (broad stretch-amide and lactam).

### Example 7

### Synthesis of 2'-OMe-Analog, 35

### Step 32: Synthesis of compound 33

A solution of 218 mg of the tosylate, **25**, in 2.5 ml of DMF at 0 °C was treated with 0.095 ml of DBU, followed by 0.15 ml of benzyloxy methyl chloride under argon and stirred for 1.5 h. The reaction mixture was quenched with 2 ml of H₂O and stirred for 20 min. after which it was concentrated to dryness. The residue was taken in 150 ml of EtOAc and 50 ml of H₂O. The organic layer was separated and washed sequentially with 25 ml of 1N HCl, 50 ml of H₂O, 50 ml of brine and then dried over Na₂SO₄ and concentrated to give the crude product which was chromatographed using 49:1 CH₂Cl₂:MeOH to give 250 mg of the pure product. Confirmed by ¹H-NMR (ppm) 5.80 (1H, Anomeric H-1), 5.41-5.53 (3H, H-2 & NCH₂O), 4.67 (2H, CH₂Ph), 3.67 (3H, COOCH₃), 2.43 (3H, tosyl-CH₃), 2.09 (3H, OCOCH₃), 1.90 (3H, thymine-CH₃).

### Step 33 : Synthesis of compound 34

A solution of 245 mg of compound **33** in 4.0 ml of THF was treated with 2.4 ml of 0.5N NaOH at room temperature and stirred for 3 h. It was then neutralized with 1.3 ml of 1N HCl and diluted with 10 ml of H₂O and lyophilized to give 220 mgs of the crude hydroxy acid which was used without further purification. Confirmed by ¹H-NMR(ppm) 5.65 (1H, Anomeric H-1), 5.33-5.34 (2H, NCH₂O), 4.59(2H, CH₂Ph), 4.26 & 4.27 (1H, H-2), 2.40 (3H, tosyl-CH₃), 1.82 (3H, thymine-CH₃); (+)FAB-MS (M+1) 575; IR(cm⁻¹) 1708 (acid stretch) 1661 (broad stretch-amide and lactam).

### Step 34: Synthesis of compound 35

A suspension of 50 mgs of the hydroxy acid, **34**, in 1.5 ml of THF and 0.11 ml of DMF was cooled to -10 °C and treated with 8 mgs of NaH and stirred for 20 min. Then it was treated with 0.06 ml of MeI and continued stirring for 6 h. during which the temperature had risen up to 20 °C. During the work up it was cooled to 0 °C and diluted with 15 ml of EtOAc and quenched with 2 ml of sat. NH₄Cl and stirred for 15 min. It was further diluted with 50 ml of EtOAc and 10 ml of H₂O. The organic layer was separated and washed with 25 ml of 0.01N HCl, 25 ml of brine and dried over Na₂SO₄. After removing the EtOAc the crude product was chromatographed over silica gel using 7:1:0.5:0.5 EtOAc/MeCN/MeOH/H₂O to give 26 mg of the pure methyl ether-acid. Confirmed by ¹H-NMR(ppm) 5.88 (1H, Anomeric H-1), 5.48-5.49 (2H, NCH₂O), 4.71 (2H, CH₂Ph), 3.94 & 3.96 (1H, H-2), 3.54 (3H, OCH₃), 2.45 (3H, tosyl-CH₃), 1.91 (3H, thymine-CH₃).

### Step 35 : Synthesis of compound 36

A solution of 37.0 g of the methyl ester **5** and 23.5 ml of 4-penten-1ol in 150 ml of ClC₂H₄Cl at 0 °C treated with 37 ml of TMSOTf in 100ml of ClC₂H₄Cl under argon and stirred for 30 min. The ice bath was removed and the reaction was allowed to stir for an additional 1 h. The reaction mixture was then added with 200 ml of sat. NaHCO₃ (aq.) at 0 °C. The solution was stirred for another 30 min. It was then diluted with 200 ml CH₂Cl₂. The organic layer was separated and washed with 100 ml brine, dried over Na₂SO₄ and concentrated to give the crude product. This crude product was chromatographed with hexane/ethyl acetate to give 25.7 g of pure compound **36**. Confirmed by (+)FAB-MS (M+1) 397; CH calc for C₁₉H₂₄O₇S, C=57.56, H=6.10, found C=57.32, H=6.09.

### Step 36: Synthesis of compound 37

A suspension of 25.7 g of compound **36** in MeOH/H₂O/Et₃N (500ml/400ml/100ml) was stirred at room temperature for 3 h. The reaction mixture was then concentrated to dryness on a rotary evaporator to give 28 g of the hydroxy acid. The resulting hydroxy acid was dissolved in 350 ml of dry THF at 0°C and treated with 4.7 g of NaH rinsed in 100ml THF and stirred for 30 min. Then it was treated with 60 ml of MeI and continued stirring for 2 h. during which the temperature had risen up to room temperature. During the work up it was cooled to 0 °C and diluted with 400 ml of EtOAc and 200 ml of water. After evaporattion off the THF, the solution was acidified with 1.0 N HCl to pH 5, and then extracted with 500 ml of EtOAc three times. The organic layer was separated and washed with 250 ml of brine and dried over Na₂SO₄. After removing the EtOAc the crude product was taken in 220 ml of DMF. This solution was then treated with 9.95 g of K₂CO₃ and 7.5 ml of benzyl bromide. The reaction was allowed to stir for 3h. During the work up it was concentrated to dryness and the residue was partitioned between 1000 ml of EtOAc and 200 ml of H₂O. The organic layer was separated and washed with brine after which it was dried over Na₂SO₄ and concentrated to give the crude product. This crude product was chromatographed with hexane/Ethyl Acetate to give 25.1 g of pure compound **37**. Confirmed by FAB (M) 518.

### Step 37: Synthesis of Compound 38

A solution of 3.8 g of pentenyl glycoside **37** in 70 mL of CH₃CN was stirred with 1.81 g of N-iodosuccinimide and 4.68 g of trimethylsilyl-N-6-benzoyl cytosine for 15 min. It was then treated with a solution of 2.8 ml of TMSOTf in 25 ml of CH₃CN over a period of 10 minutes. After 3 h, the reaction was diluted with 300 ml of EtOAc and 50 ml of 10% aqueous Na₂S₂O₃. The reaction mixture was then filtered over a celite pad. The filter cake was washed with 200 ml of EtOAC and the combined filtrates were washed with 25 ml of 5% NaHCO₃ followed by 25 ml of brine, dried over Na₂SO₄ and concentrated to dryness. This crude product was chromatographed with CH₂Cl₂/MeOH to give 2.9 g of compound **38**. The product was recrystallized from ethanol to give 2.2 g of pure **38** as a white solid. Confirmed by (+)FAB-MS (M+1) 648; CHN calc for C₃₃H₃₃N₃O₉S, C=61.20, H=5.14, N=6.49, found C=60.86, H=5.12, N=6.39.

### Step 38: Synthesis of Compound 39

A solution of 2.1 g of the Tosylnucleoside **38** and 0.95 g of Lithium azide and 32 mL DMF was stirred at 80° for 4h. The reaction mixture was evaporated and then partitioned between EtOAC and water. The organic layer was dried over Na₂SO₄, evaporated and chromatographed with CH₂Cl₂/MeOH to give 265 mg of the azide **39**. Confirmed by (+) FAB MW 519.

### Step 39: Synthesis of compound 40

A mixture of 1.68 g of the azide **39** in 40 ml of dioxane and 20 ml of water was treated with 0.134 g of 20 % Pd(OH)₂ and stirred under H₂ atmosphere at STP conditions. The reaction mixture was stirred for 2.5 h after which it was filtered over a celite pad. The filter cake was washed with 100 ml of dioxane/water (1:1) and the combined filtrates were concentrated to give the desired amino acid. A solution of the resulting amino acid and 1.39 g of N-(9-fluorenylmethoxycarbonyloxy)-succinmide in 90 ml of DMF/H₂O(1:1) was cooled to 0 °C. It was then treated with 0.8 g of NaHCO₃ and stirred for 4 h at room temperature. During the work up the reaction mixture was concentrated to dryness on a rotary evaporator at 35 °C. The residue was taken into 50 ml of H₂O and 500 ml of EtOAc. The aqueous layer was acidified with 1.0 N HCl to pH 4 and then extracted with 500 ml of EtOAc three times. After removing the EtOAc the crude product was purified on a silica gel column with CH₂Cl₂/MeOH to give 1.5 g of the pure compound **40** as a white solid. Confirmed by (+)FAB (M+1) 625.

### Step 40: Synthesis of compound 41

To a solution of the amine **13** (1 mmol, see Scheme 3) and aldehyde in MeOH containing 1% acetic acid (10mL) is added NaBH₃CN (1 mmol) over 40 min at room temperature. After the reaction is completed and quenched, one would evaporate the solution to dryness and partition the residue between EtOAC and sat. NaHCO₃. The organic layer would then be separated and washed with brine after which it would be dried over Na₂SO₄ and concentrated to give the crude product.

### Step 41: Synthesis of compound 42

A solution of 100 mg of amine **41** in 2 ml of acetonitrile is sequentially treated with 0.07 ml of triethylamine and 88 mg of FMOC-Cl and the reaction mixture is stirred at room temperature for several hour. One would then concentrate the reaction mixture to dryness. The residue would be redissolved in 2 ml of CH₂Cl₂ and loaded on a silica gel column and eluted using hexane/EtOAc to give pure product.

Steps 42-44 would follow the same experimental procedure as described in steps 14-16 (scheme 3).

### Example 8

### Solid Phase Synthesis of Oligomer

The oligomer (H-T-C-T-C-T-C-T-C-C-T-T-C-T-lys-NH₂) [SEQ ID NO:3] was synthesized by solid-phase method using Fmoc-protected monomers on a benzhydrylamine (BHA) resin. The resin was swollen, neutralized, washed, and then coupled with ρ-[(R,S)-a-[1-(9H-fluoren-9-yl)-methoxyformamido]-2,4-dimethoxybenzyl]-phenoxyacetic acid or 5-(9-Fmoc-anilnoxanthen-3-oxy)valeric acid. After removal of the Fmoc group from the resin bound Rink (or Xal) linker by treatment with 20% piperidine in N,N-dimethylformamide (DMF), the resulting amine was acylated with Fmoc-N-ε-t-butyloxycarbonyl- 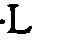 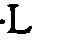 -lysine to obtain Fmoc-lys(Boc)-Rink (or Xal)-BHA resin. The first monomer, Fmoc thymine monomer (5 equiv.), was coupled to the Fmoc deprotected resin for 15 min using 4.5 equiv. of the coupling reagent HATU [O-(7-azabenzotriazol-1-yl)-1,1,3,3-tetramethyluronium hexafluorophosphate], 10 equiv. of the base N,N-diisopropylethylamine (DIPEA) in DMF. After completion of coupling and washing, the temporary Fmoc protecting group was removed with a deblocking solution consisting of 2% 1,8-diazabicyclo[5.4.0]undec-7-ene (DBU) in DMF (1 min, 7 min, then 7 min). Following deprotection, the resin was washed with methylene chloride (CH₂Cl₂) and DMF. Subsequent monomers were coupled to the resin in a manner analogous to the first monomer, Fmoc thymine monomer. Optional capping was carried out with 5% acetic anhydride in DMF. After assembly of the sequence was complete, the resin was washed and the terminal Fmoc group was removed. The resin was then washed with DMF, methylene chloride, and methanol, and dried *in vacuo* in preparation for cleavage. The fully protected oligomer was cleaved with 50% trifluoroacetic acid in CH₂Cl₂ and purified using reversed-phase HPLC. The final deprotection was carried out by treating the oligonucleotide with concentrated ammonium hydroxide for four hours at room temperature. An equal volume of 20% ethylenediamine/phenol was then added to the reaction and mixed for 1 additional hour. The reaction was terminated by evaporation of the sample in the speedvac concentrator, yielding a mixture that was applied to a reversed-phase HPLC column for purification. The purified oligomer was homogeneous as measured by analytical HPLC and the expected molecular weight was confirmed by MALDI-TOF mass spectrometry [found (calcd), 3711.7 (3712.1)]. The oligomerization of the 2'-OMe-analog was also carried out by solid-phase method using the similar protocol.

Similarly, an oligomer (H-T-C-T-C-T-C-T-C-C-T-T-C-T-NH₂) [SEQ ID NO:3] can be prepared by the above procedure, with the exception of omitting the lysine coupling step. In other words, the first monomer is coupled directly to the linker [i.e. H-Rink (or Xal)-BHA resin]. By this general procedure, oligomers of this invention can be prepared.

### Example 9

### Hybridization Properties of Oligomer Thermal Melting Studies

Absorbance versus temperature curves were measured at 260 nm using a Cary 3 spectrophotometer equipped with an electrothermal temperature controller and interfaced to an IBM PS2/50Z computer. Oligonucleotide concentration was 1.4 µM and the buffer contained 100 mM NaCl 10 mM sodium phosphate, and 0.1 mM EDTA, pH 7. Tm values were determined from the maxim of first derivative plots using the Reducep program (Koerber, S.C.; Fink, A.L. *Analytical Biochemistry* **1987**, *165*, 75-87) which utilizes the Savitzky-Golay algorithm (Savitzky, A.; Golay, M.J.E. *Analytical Chemistry* **1964**, *36*, 1627-39) thermodynamic constants were obtained from fits of data to a two-state model with linear sloping baselines (Petersheim, M.; Turner, D.H. *Biochemistry* **1983**, *22*, 256-263).

### Conditions: 100 mM NaCl, 10 mM sodium phosphate, 0.1 mM EDTA, pH 7. Melting Temperatures (Tm's) in °C

| | 5'-TCTCTCTCCTTCT [SEQ ID NO:3] | | |
|---|---|---|---|
| | Phosphorothioate | R-PNA | PNA |
| 5'-AGAAGGAGAGAGA [SEQ ID NO:2] | 46.1 | 41.0 | 72.1, 85.1 |
| Antiparallel RNA | (45.9) | (40.5) | (67.7) |
| 5'-AGAGAGAGGAAGA [SEQ ID NO: 1] | 27.7 | 26.0 | 77.8, >90 |
| Parallel RNA | (27.8) | (25.5) | (53.2) |
| 5'-AGAAGGAGAGAGA [SEQ ID NO:2] | 30.2 | 28.6 | (50.7), 67.9 |
| Antiparallel DNA | (30.3) | (27.4) | (46.6) |
| ( ) denotes Tm's from the ramp down measurements | | | |

As shown in this Table, the binding properties of the new oligomer (R-PNA) are very similar to that of phosphorothioates. They have similar binding affinity, polarity of binding and a preference of binding to RNA over DNA. By comparison, the Danish-PNA sequence showed much stronger binding affinity. However, it also exhibited very significant hysteresis and a poor discrimination of the less favorable bindings to DNA and parallel strand of RNA. The extraordinary binding affinity of Danish-PNA's may therefore be an disadvantage due to poor sequence specificity.

The Tm of the duplex between the 2'-OMe derivative of R-PNA (same sequence) and its complementary antiparallel RNA was found to be 43.7°C.

### Example 10

### Base-Pairing Specificity of Oligomer Thermal Melting Studies

### Conditions: 100 mM NaCl, 10 mM sodium phosphate, 0.1 mM EDTA, pH 7. Melting Temperatures (Tm's) in °C

| | 5'-TCTCTCT*C*CTTCT [SEQ ID NO:3] | |
|---|---|---|
| | Phosphorothioate | R-PNA |
| 5'-AGAAG*G*AGAGAGA [SEQ ID NO:2] | 46.1 | 41.0 |
| 5'-AGAAG*A*AGAGAGA [SEQ ID NO:4] | 30.0 | 25.7 |
| 5'-AGAAG*C*AGAGAGA [SEQ ID NO:5] | 24.1 | 21.2 |
| 5'-AGAAG*U*AGAGAGA [SEQ ID NO:6] | N.A. | 21.5 |

The results from this Table clearly illustrates that the binding of the new R-PNA series follows the Watson-Crick's complementary base-pairing rules, as the C-A, C-C and C-U mismatches showed the expected big drop in binding affinity.

### Example 11

### Cell-based Antisense assay

Antisense molecules have been demonstrated to reduce levels of the enzyme tyrosinase in melanocytes by the methods described below. These antisense molecules have potential utility for treating several diseases of hyperpigmentation (hypermelanosis) including, but not limited to café au lait macules, nevus spilus, post inflammatory mealnosis (exanthems, drug eruptions) and scleroderma.

A commonly used line of mouse melanoma cells (e.g. B-16) is raised in cell culture plates using Dulbecco's Modified Eagle's Medium supplemented with 10% fetal bovine serum and 50 µg/ml gentamicin. To initiate an experiment, antisense and control oligonucleotides are added to low density (i.e. sub-confluent) cell cultures, then treatment is continued for several days. Cells are then rinsed with saline, collected by scraping, then cells are extracted and analyzed for levels of protein by the Bradford method (Bradford, M.M. *Anal. Biochem*. **1976**, *72*: 248).

Levels of the target enzyme tyrosinase from a constant amount of extracted protein is measured essentially as described by a published procedure (Pomerantz, S. H. *J. Biol. Chem*. **1966**, *241*: 161). Briefly, extracts are incubated with tyrosine and the cofactor DOPA (3,4-dihydroxyphenylalanine.) The tyrosine is tritium labeled at the 3 and 5 positions such that tyrosinase activity is measured by the amount of tritiated water that is produced. Tritiated water is quantitated by liquid scintillation counting. The purpose of the tyrosinase assay is to establish the potency of each antisense molecule by allowing us to calculate the concentration necessary to cause 50% inhibition of tyrosinase levels.

To control for effects due to nonspecific toxicity, melanoma cells are seeded into 96-well plates at low density, then allowed to grow in the presence of antisense and control oligonucleotides for several days. Cell proliferation is assayed by the widely used tetrazolium dye procedure (Mosmann, T. *J. Immunol. Meth*. **1983**,*65*, 55). The concentration of each oligonucleotide producing 50% inhibition of growth rate is compared to the concentration of that oligonucleotide which produces 50% inhibition of tyrosinase level. Antisense effects are indicated by the following conditions: a) active oligonucleotides are complementary in sequence to the target messenger RNA, b) sense and mismatched controls are less active than fully matched antisense oligonucleotides, c) tyrosinase activity is inhibited by concentrations of antisense oligonucleotides which do not suppress cell growth.

## Claims

1. An oligomer having the structure: 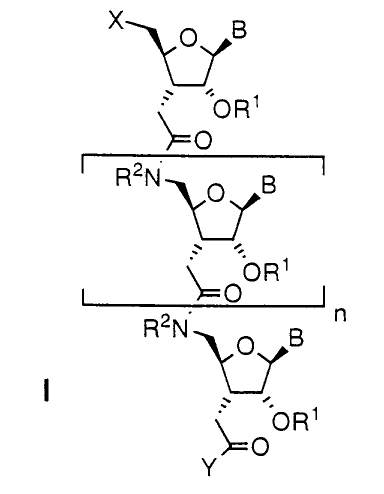 wherein R¹ is H, C₁₋₄-alkyl, C₁₋₁₈-acyl or hydroxy-lower alkyl; R² is H, aralkyl, or C₁₋₄ alkyl; B is a nucleoside base residue selected from the group consisting of adenine, cytosine, guanine, thymine, uracil; X is H, OR¹, NHR² or NH-acyl; Y is OR² or NHR²; and n is a number from 5 through 25, and salts thereof.

2. The oligomer according to claim 1 wherein R² is H and X is H, OR¹ or NHR².

3. The oligomer according to claim 1 or claim 2 wherein R¹ is methyl, acetyl or hydroxyethyl.

4. The oligomer according to claim 1 wherein R¹ and R² each are H.

5. The oligomer according to claim 1 wherein R¹ is methyl and R² is H.

6. The oligomer according to any one of of claims 1-5 wherein n is a number from 6 through 13.

7. The oligomer according to claim 1 wherein X is NH₂ or NHacyl, Y is OH or NH₂, R¹ is H or acetyl, R² is H and n is 6-13.

8. An oligomer having the structure: 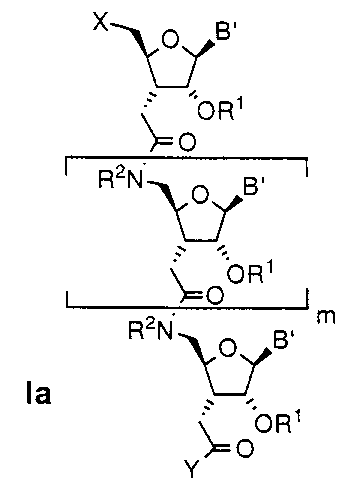 wherein R¹ is H, C₁₋₄-alkyl, C₁₋₁₈-acyl or hydroxy-lower alkyl; R² is H, aralkyl, or C₁₋₄-alkyl; B' is a suitably protected nucleoside base residue from the group consisting of adenine, cytosine, guanine, thymine, uracil; X is H, OR¹, NHR² or NH-acyl; Y is OR² or NHR²; and m is a number from 0 through 5, and salts thereof.

9. The oligomer according to claim 8 wherein m is 0, 1 or 2.

10. A compound having the formula: 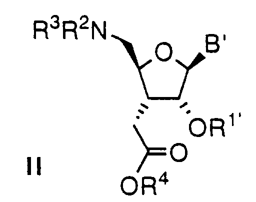 wherein R^{1'} is a hydroxy protecting group, R² is H, aralkyl or (C₁-C₄)alkyl, R³ is an amino protecting group; and R⁴ is H or an acyl protecting group; and B' is a suitably protected nucleoside base residue.

11. The compound according to claim 10 wherein B' is a N-acetylcytosine, N-benzoylcytosine, N-benzoyladenine, thymine, uracil, N-acetylguanine, or N-isobutyrylguanine residue.

12. The compound according to claim 10 or claim 11 wherein R^{1'} is C₁₋₄-alkyl, C₁₋₁₈-acyl, hydroxy-lower alkyl, or benzyl.

13. The compound according to any one of claims 10-12 wherein R³ is 9-fluorenylmethoxycarbonyl, tert-butyloxycarbonyl, benzyloxycarbonyl, allyloxycarbonyl; triphenylmethyl, or 4,4'-dimethoxytrityl.

14. The compound according to any one of claims 10-13 wherein R⁴ is tert-butyl, C₁₋₄-alkyl, benzyl, phenyl, or 2-trimethylsilylethyl.

15. A compound having the formula: 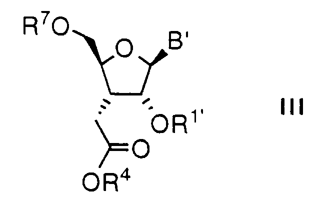 wherein R⁷ is a hydroxy protecting group; R⁴ is H, or an acyl protecting group; R^{1'} is a hydroxy protecting group; and B' is a suitably protected nucleoside base residue.

16. A medicament containing a compound according to any one of claims 1-7, and a therapeutically inert carrier material.

17. A medicament which decreases the production of a target protein in a cell, which medicament comprises an oligomer according to any one of claims 1-7 in an amount effective to bind to the mRNA sequence encoding the target protein in said cell and decrease production of said protein, and a pharmaceutically acceptable carrier.

18. A process for the manufacture of a medicament, especially for decreasing the production of a target protein in a cell, which process comprises mixing an oligomer according to any one of claims 1-7 or a pharmaceutically acceptable salt thereof with a therapeutically inert carrier material and bringing the mixture into a galenical administration form.

19. An oligomer according to any one of claims 1-7, for use as a therapeutically active compound, especially for decreasing the production of a target protein in a cell.
